# EUROPÄISCHE PATENTSCHRIFT

(11) **EP 1 328 524 B1**
(45) Veröffentlichungstag und Bekanntmachung des Hinweises auf die Patenterteilung: **12.12.2012**
(21) Anmeldenummer: 01982378.0
(22) Anmeldetag: 28.09.2001
(51) Int. Cl.: C07D 451/10, A61K 31/439, A61P 11/00

(54) **NEUE ANTICHOLINERGIKA, VERFAHREN ZU DEREN HERSTELLUNG UND DEREN VERWENDUNG ALS ARZNEIMITTEL**
NOVEL ANTICHOLINERGICS, METHOD FOR THE PRODUCTION THEREOF AND USE THEREOF AS MEDICAMENTS
NOUVEAUX ANTICHOLINERGIQUES, LEURS PROCEDES DE PRODUCTION ET LEUR UTILISATION EN TANT QUE MEDICAMENTS

(30) Priorität: 14.10.2000 DE 10050995
(43) Veröffentlichungstag der Anmeldung: 23.07.2003
(73) Patentinhaber: Boehringer Ingelheim Pharma GmbH & Co.KG, 55216 Ingelheim am Rhein (DE)
(72) Erfinder: MEISSNER, Helmut, 55218 Ingelheim (DE); MORSCHHÄUSER, Gerd, 88400 Biberach (DE); PIEPER, Michael, Paul, 88400 Biberach (DE); POHL, Gerald, 88400 Biberach (DE); REICHL, Richard, 55435 Gau-Algesheim (DE); SPECK, Georg, 55218 Ingelheim am Rhein (DE)
(86) Internationale Anmeldenummer: PCT/EP2001/011243
(87) Internationale Veröffentlichungsnummer: WO 2002/032898

(56) Entgegenhaltungen:
- EP-A- 0 513 533
- EP-A- 0 519 831
- WO-A-92/16528
- DE-A- 2 540 633
- JP-A- 06 200 202
- US-A- 3 826 836
- US-A- 5 977 115
- DATABASE CA [Online] CHEMICAL ABSTRACTS SERVICE, COLUMBUS, OHIO, US; ZAKHAROVA, N. A. ET AL: "Esters of tropine, 1-(diethylamino)-2-propanol, and.beta.-(diethylamino)ethanol" retrieved from STN Database accession no. 67:73724 XP002193560 & ZH. ORG. KHIM. (1967), 3(6), 1128-36,
- DATABASE CA [Online] CHEMICAL ABSTRACTS SERVICE, COLUMBUS, OHIO, US; EMRAN, ALI M. ET AL: "Fluorinated tropanyl esters for application with PET" retrieved from STN Database accession no. 124:111207 XP002205413 & CHEM. VIEWS IMAGING CENT., [PROC. AM. CHEM. SOC. SYMP.] (1995), MEETING DATE 1993, 485-96. EDITOR(S): EMRAN, ALI M. PUBLISHER: PLENUM, NEW YORK, N. Y.,
- OHWADA, TOMOHIKO ET AL: "Superacid-catalyzed electrocyclization of diphenylmethyl cations to fluorenes. Kinetic and theoretical revisit supporting the involvement of ethylene dications" JOURNAL OF THE AMERICAN CHEMICAL SOCIETY (1998), 120(19), 4629-4637, XP002205554
- DATABASE CA [Online] CHEMICAL ABSTRACTS SERVICE, COLUMBUS, OHIO, US; BROWN, B. T. ET AL: "Morphactin-like activity of benzilate esters in Arabidopsis thaliana" retrieved from STN Database accession no. 79:133605 XP002205460 & PLANT GROWTH SUBST., PROC. INT. CONF., 7TH (1972), MEETING DATE 1970, 318-23. EDITOR(S): CARR, DENIS J. PUBLISHER: SPRINGER, NEW YORK, N. Y.,
- CANNON: "Esters of benzylic acids and congeners having potential psychotomimetic activity" JOURNAL OF ORGANIC CHEMISTRY., Bd. 25, 1960, Seiten 959-962, XP002205458 AMERICAN CHEMICAL SOCIETY. EASTON., US ISSN: 0022-3263

## Beschreibung

Die vorliegende Erfindung betrifft neue Anticholinergika der allgemeinen Formel **1** 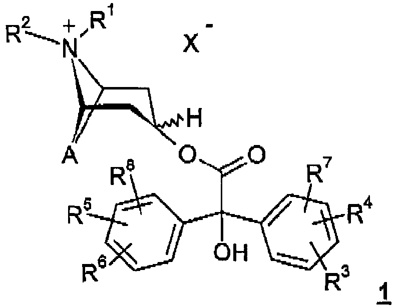 worin A, X⁻ und die Reste R¹, R², R³, R⁴, R⁵, R⁶, R⁷ und R⁸ die in den Ansprüchen und in der Beschreibung genannten Bedeutungen haben können, Verfahren zu deren Herstellung sowie deren Verwendung als Arzneimittel.

### Hintergrund der Erfindung,

Anlicholinergika können bei einer Vielzahl von Erkrankungen therapeutisch sinnvoll eingesetzt werden. Hervorzuheben sind hier beispielsweise die Therapie von Asthma oder COPD (chronic obstructive pulmonary disease = chronisch obstruktive Lungenerkrankung). Zur Therapie dieser Erkrankungen werden durch die WO 92/16528 Anticholinergika vorgeschlagen, die ein Scopin-, Tropenol- oder auch Tropin-Grundgerüst aufweisen.

Die der WO 92/16528 zugrunde liegende Aufgabe zielt auf die Bereitstellung von anticholinerg wirksamen Verbindungen, die durch eine lang andauernde Wirksamkeit gekennzeichnet sind, Zur Lösung dieser Aufgabe werden durch die WO 92/16528 unter anderem Benzilsäureester des Scopins, Tropenols oder auch Tropins offenbart.

Zur Therapie chronischer Erkrankungen ist es häufig wünschenswert, Arzneimittel mit einer längeren Wirkungsdauer bereitzustellen. Hierdurch kann in der Regel gewährleistet werden, daß die zur Erzielung des therapeutischen Effekts erforderliche Konzentration des Wirkstoffs im Organismus über einen längeren Zeitraum gegeben ist, ohne eine allzu häufige, wiederholte Gabe des Arzneimittels durchführen zu müssen. Die Applikation eines Wirkstoffs in längeren zeitlichen Abständen trägt im übrigen in hohem Maße zum Wohlbefinden des Patienten bei. Besonders wünschenswert ist die Bereitstellung eines Arzneimittels, welches therapeutisch sinnvoll durch einmalige Applikation pro Tag (Einmalgabe) eingesetzt werden kann. Eine einmal pro Tag erfolgende Anwendung hat den Vorteil, daß der Patient sich relativ schnell an die regelmäßige Einnahme des Medikaments zu bestimmten Tageszeiten gewöhnen kann.

Um als einmal täglich anwendbares Medikament zum Einsatz kommen zu können, sind an den zu applizierenden Wirkstoff besondere Anforderungen zu stellen. Zunächst sollte der nach Gabe des Arzneimittels erwünschte Wirkungseintritt relativ schnell erfolgen und im Idealfall über einen sich daran anschließende längeren Zeitraum eine möglichst konstante Wirksamkeit aufweisen. Andererseits sollte die Wirkdauer des Arzneimittels einen Zeitraum von etwa einem Tag nicht wesentlich überschreiten. Im Idealfall zeigt ein Wirkstoff ein derart geartetes Wirkungsprofil, daß sich die Herstellung eines einmal täglich applizierbaren Arzneimittels, welches den Wirkstoff in therapeutisch sinnvollen Dosen enthält, gezielt steuern läßt.

Es wurde gefunden, daß die in der WO 92/16528 offenbarten Benzilsäureester des Scopins, Tropenols oder auch Tropins diesen erhöhten Anforderungen nicht genügen. Sie sind aufgrund ihrer extrem langen Wirkungsdauer, die den vorstehend genannten Zeitraum von etwa einem Tag deutlich überschreiten, nicht als Einmalgabe pro Tag therapeutisch nutzbar.

Es ist daher Aufgabe der vorliegenden Erfindung, neue Anticholinergika bereitzustellen, die aufgrund ihres Wirkungsprofils die Herstellung eines einmal täglich applizierbaren Arzneimittels erlauben. Es ist ferner Aufgabe der Erfindung, Verbindungen bereitzustellen, die durch einen relativ raschen Wirkungseintritt gekennzeichnet sind. Es ist des weiteren Aufgabe der Erfindung, Verbindungen bereitzustellen, die nach raschem Wirkungseintritt über einen sich daran anschließenden längeren Zeitraum eine möglichst konstante Wirksamkeit aufweisen. Ferner ist es Aufgabe der Erfindung, Verbindungen bereitzustellen, deren Wirkdauer einen Zeitraum von etwa einem Tag bei therapeutisch sinnvoll einsetzbaren Dosierungen nicht wesentlich überschreitet. Schließlich ist es Aufgabe der Erfindung, Verbindungen bereitzustellen, die ein Wirkungsprofil aufweisen, welches eine gute Steuerbarkeit des therapeutischen Effekts (das heißt volle therapeutische Wirkung ohne Nebenwirkung durch Kumulation der Substanz im Organismus) gewährleistet.

### Detaillierte Beschreibung der Erfindung

Überraschenderweise wurde gefunden, daß die vorstehend genannten Aufgaben durch Verbindungen der allgemeinen Formel **1** gelöst werden, in denen wenigstens einer der Reste R³, R⁴, R⁵, R⁶, R⁷ und R⁸ nicht Wasserstoff bedeutet.

Dementsprechend zielt die vorliegende Erfindung auf Verbindungen der allgemeinen Formel **1** 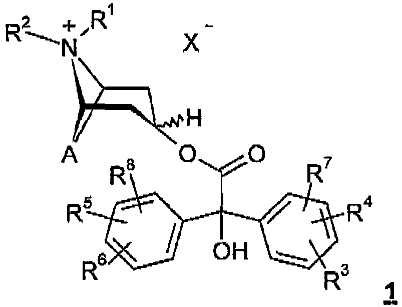 worin
- A: ein zweibindiger Rest ausgewählt aus der Gruppe bestehend aus 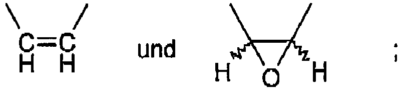
- X⁻: ein einfach negativ geladenes Anion,
- R¹ und R²: C₁-C₄-Alkyl, welches gegebenenfalls durch Hydroxy oder Halogen substituiert sein kann;
- R³, R⁴, R⁵, R⁶, R⁷ und R⁸,: gleich oder verschieden, Wasserstoff oder Fluor, mit der Maßgabe, daß wenigstens einer der Reste R³, R⁴, R⁵, R⁶, R⁷ und R⁸ nicht Wasserstoff ist, bedeuten.

Bevorzugt sind Verbindungen der allgemeinen Formel **1**, worin
- A: ein zweibindiger Rest ausgewählt aus der Gruppe bestehend aus 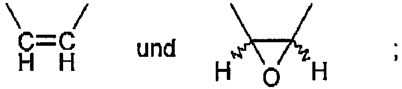
- X⁻: ein einfach negativ geladenes Anion ausgewählt aus der Gruppe Chlorid, Bromid, Methylsulfat, 4-Toluolsulfonat und Methansulfonat, bevorzugt Bromid,
- R¹ und R²: gleich oder verschieden ein Rest ausgewählt aus der Gruppe bestehend aus Methyl, Ethyl, n-Propyl und iso-Propyl, der gegebenenfalls durch Hydroxy oder Fluor substituiert sein kann, bevorzugt unsubstituiertes Methyl;
- R³, R⁴, R⁵, R⁶, R⁷ und R⁸,: gleich oder verschieden, Wasserstoff oderFluor, , mit der Maßgabe, daß wenigstens einer der Reste R³, R⁴, R⁵, R⁶, R⁷ und R⁸ nicht Wasserstoff ist, bedeuten.

Besonders bevorzugt sind Verbindungen der allgemeinen Formel **1**, worin
- A: ein zweibindiger Rest ausgewählt aus der Gruppe bestehend aus 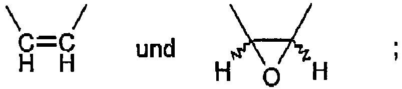
- X⁻: Bromid;
- R¹ und R²: gleich oder verschieden Methyl oder Ethyl, bevorzugt Methyl;
- R³, R⁴, R⁵, R⁶, R⁷ und R⁸,: gleich oder verschieden, Wasserstoff oder Fluor, mit der Maßgabe, daß wenigstens einer der Reste R³, R⁴, R⁵, R⁶, R⁷ und R⁸ nicht Wasserstoff ist, bedeuten.

Erfindungsgemäß von besonderer Bedeutung sind Verbindungen der allgemeinen Formel **1**, worin
- A: ein zweibindiger Rest ausgewählt aus der Gruppe bestehend aus 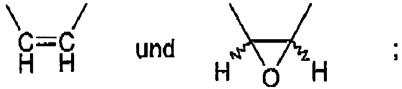
- X⁻: Bromid;
- R¹ und R²: gleich oder verschieden Methyl oder Ethyl, bevorzugt Methyl;
- R³, R⁴, R⁵, R⁶, R⁷ und R⁸,: gleich oder verschieden, Wasserstoff oder Fluor, mit der Maßgabe, daß wenigstens einer der Reste R³, R⁴, R⁵, R⁶, R⁷ und R⁸ nicht Wasserstoff ist, bedeuten.

Von erfindungsgemäß herausragender Bedeutung sind die Verbindungen der allgemeinen Formel **1**, worin
- A: ein zweibindiger Rest ausgewählt aus der Gruppe bestehend aus 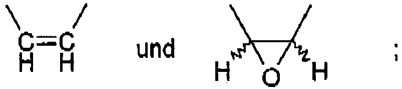
- X⁻: Bromid;
- R¹ und R²: Methyl;
- R³, R⁴, R⁵, R⁶, R⁷ und R⁸,: gleich oder verschieden, Wasserstoff oder Fluor mit der Maßgabe, daß wenigstens einer der Reste R³, R⁴, R⁵, R⁶, R⁷ und R⁸ nicht Wasserstoff ist, bedeuten.

Gegenstand der Erfindung sind die jeweiligen Verbindungen der Formel **1** gegebenenfalls in Form der einzelnen optischen Isomeren, Mischungen der einzelnen Enantiomeren oder Racemate.

In den Verbindungen der allgemeinen Formel **1** können die Reste R³, R⁴, R⁵, R⁶, R⁷ und R⁸, sofern sie nicht Wasserstoff bedeuten, jeweils ortho, meta oder para bezüglich der Verknüpfung zur "-C-OH"-Gruppe angeordnet sein. Sofern keiner der Reste R³, R⁴, R⁵, R⁶, R⁷ und R⁸ Wasserstoff bedeutet sind R³und R⁵ bevorzugt in para-Position und R⁴, R⁶, R⁷ und R⁸ bevorzugt in ortho- oder meta-Position, besonders bevorzugt in meta-Position verknüpft. Besonders bevorzugt sind Verbindungen der Formel **1**, in denen die Reste R⁷ und R⁸ Wasserstoff bedeuten. Sofern in diesem Fall einer der Reste R³ und R⁴ und einer der Reste R⁵ und R⁶ Wasserstoff bedeutet, ist der jeweils andere Rest bevorzugt in meta- oder para-Position, besonders bevorzugt in para-Position verknüpft. Sofern keiner der Reste R³, R⁴, R⁵, R⁶, R⁷ und R⁸ Wasserstoff bedeutet sind erfindungsgemäß die Verbindungen der allgemeinen Formel **1** besonders bevorzugt, in denen die Reste R³, R⁴, R⁵, R⁶, R⁷ und R⁸ die selbe Bedeutung aufweisen.

Von besonderer Bedeutung sind erfindungsgemäß die Verbindungen der allgemeinen Formel **1**, in denen der Ester-Substituent am Stickstoff-Bicylus α-konfiguriert ist. Diese Verbindungen entsprechen der allgemeinen Formel **1-**α

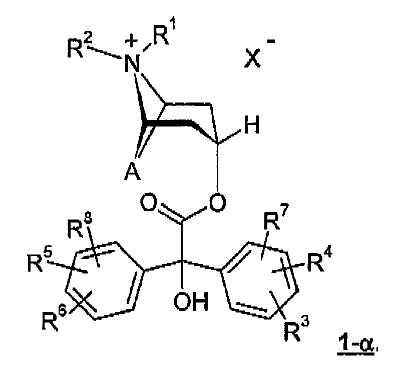

Die nachfolgenden Verbindungen sind erfindungsgemäß von besonderer Bedeutung:
- 3,3',4,4'-Tetrafluorbenzilsäuretropenolester-Methobromid;
- 3,3',4,4'-Tetrafluorbenzilsäurescopinester-Methobromid;
- 4,4'-Dichlorbenzilsäurescopinester-Methobromid; 4,4'-Difluorbenzilsäurescopinester-Methobromid;
- 3,3'-Difluorbenzilsäuretropenolester-Methobromid;
   3,3'-Difluorbenzilsäurescopinester-Methobromid;
- 4,4'-Difluorbenzilsäuretropenolester-Ethylbromid;

Als Alkylgruppen werden, soweit nicht anders angegeben, verzweigte und unverzweigte Alkylgruppen mit 1 bis 4 Kohlenstoffatomen bezeichnet. Beispielsweise werden genannt: Methyl, Ethyl, Propyl oder Butyl. Zur Bezeichnung der Gruppen Methyl, Ethyl, Propyl oder auch Butyl werden gegebenenfalls auch die Abkürzungen Me, Et, Prop oder Bu verwendet. Sofern nicht anders beschrieben, umfassen die Definitionen Propyl und Butyl alle denkbaren isomeren Formen der jeweiligen Reste, So umfaßt beispielsweise Propyl n-Propyl und iso-Propyl, Butyl umfaßt iso-Butyl, sec. Butyl und tert,-Butyl etc.

Als Alkyloxygruppen werden, soweit nicht anders angegeben, verzweigte und unverzweigte Alkylgruppen mit 1 bis 4 Kohlensfioffatomen bezeichnet, die über ein Sauerstoffatom verknüpft sind. Beispielsweise werden genannt: Methyloxy, Ethyloxy, Propyloxy oder Butyloxy. Zur Bezeichnung der Gruppen Methyloxy, Ethyloxy, Propyloxy oder auch Butyloxy werden gegebenenfalls auch die Abkürzungen MeO-, EtO-, PropO- oder BuO- verwendet. Sofern nicht anders beschrieben, umfassen die Definitionen Propyloxy und Butyloxy alle denkbaren isomeren Formen der jeweiligen Reste. So umfaßt beispielsweise Propyloxy n-Propyloxy und iso-Propyloxy, Butyloxy umfaßt iso-Butyloxy, sec, Butyloxy und tert.-Butyloxy etc. Gegebenenfalls wird im Rahmen der vorliegenden Erfindung statt der Bezeichnung Alkyloxy auch die Bezeichnung Alkoxy verwendet. Zur Bezeichnung der Gruppen Methyloxy, Ethyloxy, Propyloxy oder auch Butyloxy gelangen dementsprechend gegebenenfalls auch die Ausdrücke Methoxy, Ethoxy, Propoxy oder Butoxy zur Anwendung.

Halogen steht im Rahmen der vorliegenden Erfindung für Fluor, Chlor, Brom oder Jod. Sofern nicht gegenteilig angegeben, gilt Brom als bevorzugtes Halogen.

Die Herstellung der erfindungsgemäßen Verbindungen kann, wie nachstehend erläutert, zum Teil in Analogie zu im Stand der Technik bereits bekannten Vorgehensweisen erfolgen (Schema 1).

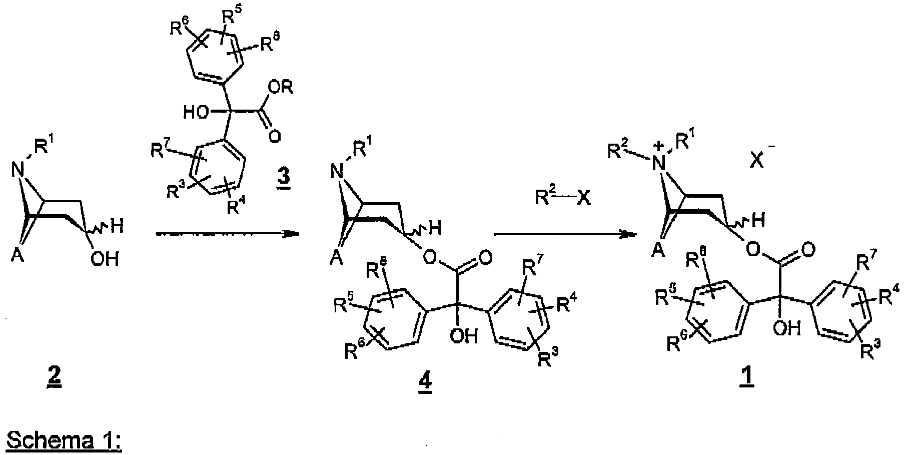

Ausgehend von den Verbindungen der Formel **2** gelingt der Zugang zu den Estern der allgemeinen Formel **4** durch Umsetzung mit den Benzilsäureestern der Formel **3**, in denen R beispielsweise für einen C₁-C₄-Alkylrest steht. Diese Umsetzung kann beispielsweise in einer Natriumschmelze bei erhöhter Temperatur, bevorzugt bei ca. 50-150°C, besonders bevorzugt bei etwa 90-100°C bei niedrigem Druck, bevorzugt bei unter 500mbar, besonders bevorzugt bei unter 75mbar durchgeführt werden. Alternativ dazu können statt der Benzilsäureester **3** auch die entsprechenden α-Chlor-Verbindungen (Cl statt OH) eingesetzt werden. In diesem Fall kann die Umsetzung in Analogie zu den in der WO 92/16528 offenbarten Synthesebeispielen, auf die an dieser Stelle voll inhaltlich Bezug genommen wird, durchgeführt werden. Die so erhaltenen Verbindungen der Formel **4** lassen sich durch Umsetzung mit den Verbindungen R²-X, in denen R² und X die vorstehend genannten Bedeutungen haben können, in die Zielverbindungen der Formel **1** überführen. Auch die Durchführung dieses Syntheseschritts kann in Analogie zu den in der WO 92/16528 offenbarten Synthesebeispielen erfolgen.

Alternativ zu der in Schema 1 dargestellten Vorgehensweise zur Synthese der Verbindungen der Formel **4** lassen sich die Derivate **4**, in denen der Stickstoffbicyclus ein Scopin-Derivat darstellt, durch Oxidation von Verbindungen der Formel **4** erhalten, in denen der Stickstoffbicyclus ein Tropenyl-Rest ist. Hierzu kann erfindungsgemäß wie folgt vorgegangen werden.
Die Verbindung **4**, in der A für -CH=CH- steht, wird in einem polaren organischen Lösemittel, bevorzugt in einem Lösemittel ausgewählt aus der Gruppe N-Methyl-2-pyrrolidon (NMP), Dimethylacetamid und Dimethylformamid, bevorzugt Dimethylformamid suspendiert und anschließend erwärmt auf eine Temperatur von ca. 30-90°C, vorzugsweise 40-70°C. Anschließend wird ein geeignetes Oxidationsmittel zugegeben und bei konstanter Temperatur 2 bis 8 Stunden, bevorzugt 3 bis 6 Stunden gerührt. Als Oxidationsmittel kommt bevorzugt Vanadiumpentoxid im Gemisch mit H₂O₂, besonders bevorzugt H₂O₂-Harnstoffkomplex in Kombination mit Vanadiumpentoxid zur Anwendung. Die Aufarbeitung erfolgt auf üblichem Wege. Die Reinigung der Produkte kann je nach Kristallisationsneigung durch Kristallisation oder Chromatographie erfolgen.

Wie in Schema 1 ersichtlich, kommt den Zwischenprodukten der allgemeinen Formel **4** eine zentrale Bedeutung zu, Dementsprechend zielt ein weiterer Aspekt der vorliegenden Erfindung auf die intermediate der Formel **4** 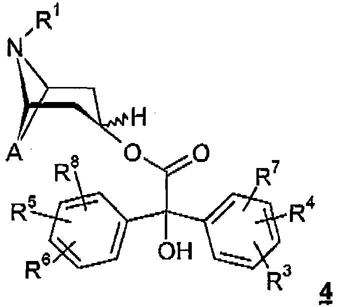 worin
- A: ein zweibindiger Rest ausgewählt aus der Gruppe bestehend aus 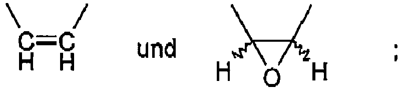
- R¹: C₁-C₄-Alkyl, welches gegebenenfalls durch Hydroxy oder Halogen substituiert sein kann;
- R³, R⁴, R⁵, R⁶, R⁷ und R⁸,: gleich oder verschieden, Wasserstoff oder Fluor , mit der Maßgabe, daß wenigstens einer der Reste R³, R⁴, R⁵, R⁶, R⁷ und R⁸ nicht Wasserstoff ist, bedeuten.

Bevorzugt sind die Intermediate der allgemeinen Formel **4**, worin
- A: ein zweibindiger Rest ausgewählt aus der Gruppe bestehend aus 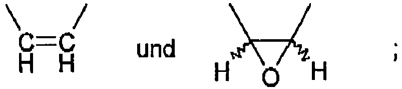
- R¹: ein Rest ausgewählt aus der Gruppe bestehend aus Methyl, Ethyl, n-Propyl und iso-Propyl, der gegebenenfalls durch Hydroxy oder Fluor substituiert sein kann, bevorzugt unsubstituiertes Methyl;
- R³, R⁴, R⁵, R⁶, R⁷ und R⁸,: gleich oder verschieden, Wasserstoff oder, Fluor, mit der Maßgabe, daß wenigstens einer der Reste R³, R⁴, R⁵, R⁶, R⁷ und R⁸ nicht Wasserstoff ist bedeuten.

Besonders bevorzugt sind die Intermediate der allgemeinen Formel **4**, worin
- A: ein zweibindiger Rest ausgewählt aus der Gruppe bestehend aus 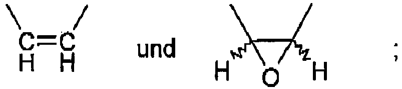
- R¹: Methyl oder Ethyl, bevorzugt Methyl;
- R3, R⁴, R⁵, R⁶, R⁷ und R⁸,: gleich oder verschieden, Wasserstoff oderFluor, mit der Maßgabe, daß wenigstens einer der Reste R³, R⁴, R⁵, R⁶, R⁷ und R⁸ nicht Wasserstoff ist, bedeuten.

Von erfindungsgemäß herausragender Bedeutung sind die Intermediate der allgemeinen Formel **4**, worin
- A: ein zweibindiger Rest ausgewählt aus der Gruppe bestehend aus 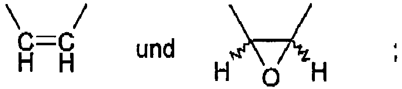
- R¹: Methyl;
- R3, R⁴, R⁵, R⁶, R⁷ und R⁸,: gleich oder verschieden, Wasserstoff oder Fluor, mit der Maßgabe, daß wenigstens einer der Reste R³, R⁴, R⁵, R⁶, R⁷ und R⁸ nicht Wasserstoff ist, bedeuten.

Wie in den Verbindungen der allgemeinen Formel **1** können auch in den Intermediaten der Formel **4** die Reste R³, R⁴, R⁵, R⁶, R⁷ und R⁸, sofern sie nicht Wasserstoff bedeuten, jeweils ortho, meta oder para bezüglich der Verknüpfung zur "-C-OH"-Gruppe angeordnet sein. Sofern keiner der Reste R³, R⁴, R⁵, R⁶, R⁷ und R⁸ Wasserstoff bedeutet sind R³und R⁵ bevorzugt in para-Position und R⁴, R⁶, R⁷ und R⁸ bevorzugt in ortho- oder meta-Position, besonders bevorzugt in meta-Position verknüpft. Besonders bevorzugt sind Intermediate der Formel **4** in denen R⁷ und R⁸ Wasserstoff bedeuten. Sofern in diesem Fall einer der Reste R³ und R⁴ und einer der Reste R⁵ und R⁶ Wasserstoff bedeutet, ist der jeweils andere Rest bevorzugt in meta- oder para-Position, besonders bevorzugt in para-Position verknüpft. Sofern keiner der Reste R³, R⁴, R⁵, R⁶, R⁷ und R⁸ Wasserstoff bedeutet sind erfindungsgemäß die Intermediate der allgemeinen Formel **4** besonders bevorzugt, in denen die Reste R³, R⁴, R⁵, R⁶, R⁷ und R⁸ dieselbe Bedeutung aufweisen.

Zur Herstellung der Zwischenprodukte der allgemeinen Formel **4** werden entsprechend substituierte Benzilsäure-Derivate der allgemeinen Formel **3** eingesetzt. Diese werden in Analogie zu im Stand der Technik bekannten Verfahren durch Umsetzung von aus den Bromide **5** in situ generierten Grignard-Reagentien mit den aromatischen α-Carbonyl-carbonsäureestern **6** erhalten,

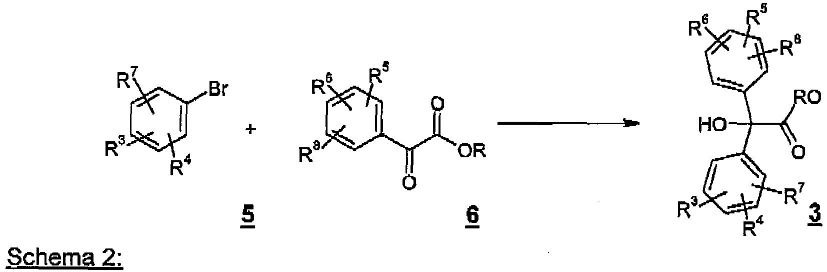

Die Reaktion wird in wasserfreien organischen Lösemitteln, bevorzugt in etherischen Lösemittel, besonders bevorzugt in einem Lösemittel ausgewählt aus der Gruppe Diethylether, Dioxan und Tetrahydrofuran (THF), wovon letzterem besondere Bedeutung zukommt, geführt. Die Generierung des Grignardreagenzes erfolgt aus den Bromiden **5** durch Umsetzung mit Mg-Spänen. Gegebenenfalls kann der Zusatz eines Reaktionsstarters wie Jod oder Dibromethan erforderlich sein. Zur Vervollständigung der Bildung des Grignard-Reagenzes kann es erforderlich sein, die Reaktion über einen Zeitraum von 0,5 bis 2 Stunden zu erwärmen, bevorzugt auf über 30°C, besonders bevorzugt auf über 50°C. Die Obergrenze des hierzu wählbaren Temperaturbereichs bestimmt sich naturgemäß aus der Siedetemperatur des verwendeten Lösemittels. Das so erhaltene Grignard-Reagenz wird dann zu einer Lösung von **6** in einem der vorstehend genannten Lösemittel langsam zugetropft. Hierbei kann bei Raumtemperatur, vorzugsweise aber bei einer Temperatur im Bereich von 0-15°C gearbeitet werden. Die Reaktion ist in der Regel nach 1 bis 4 Stunden vollständig. Die Aufarbeitung erfolgt nach üblichen Methoden. Die Reinigung der Produkte erfolgt je nach Kristallisationsneigung der Verbindungen **3** durch Kristallisation oder mittels Säulenchromatographie.
Alternativ dazu sind die Verbindungen der Formel **3** auch in Analogie zu anderen aus dem Stand der Technik bekannte Syntheseverfahren zugänglich. Sind beispielsweise geeignet substituierte Benzilsäuren bereits im Stand der Technik bekannt und kommerziell erhältlich, können die Verbindungen der Formel **3** auch direkt aus diesen durch Säure- oder Basen-katalysierte Veresterung mit den entsprechenden Alkoholen R-OH erhalten werden. Sind geeignet substituierte Benzile bereits im Stand der Technik bekannt und kommerziell erhältlich, können die Verbindungen der Formel **3** auch direkt aus diesen durch Benzilsäureumlagerung und anschließende Säure- oder Basen-katalysierte Veresterung mit den entsprechenden Alkoholen R-OH erhalten werden.

Wie aus Schema 1 ersichtlich, kommt den Benzilsäurederivaten der allgemeinen Formel 3 eine zentrale Bedeutung bei der Herstellung der Verbindungen der allgemeinen Formel **4** und somit auch **1** zu.

Die nachstehend beschriebenen Synthesebeispiele dienen der weitergehenden Illustration der vorliegenden Erfindung. Sie sind allerdings nur als exemplarische Vorgehensweisen zur weitergehenden Erläuterung der Erfindung zu verstehen, ohne selbige auf den nachfolgend exemplarisch beschriebenen Gegenstand zu beschränken.

### I. Synthese der Benzilsäurederivate der Formel 3:

### I.1.:3,3',4,4'-Tetrafluorbenzilsäureethylester 3a:.

Die Herstellung des Grignard-Reagenzes erfolgt aus 2,24 g (0,092 mol) Magnesiumspäne, einigen Körnchen Jod und 17,80 g (0,092 mol) 1-Brom-3,4-difluor-benzol in 100 ml THF bei 50° C. Nach beendeter Zugabe des Halogenids wird noch eine Stunde nachgerührt. Das so erhaltene Grignard-Reagenz wird zu 18,81 g (0,088 mol) 3,4-Difluorphenylglyoxylsäureethylester in 80 ml THF bei 10°-15° C tropfenweise zugesetzt und die erhaltene Mischung 2 Stunden bei 5° C gerührt.
Die weiße Suspension wird zur Aufarbeitung auf Eis/Schwefelsäure gegossen, mit Ethylacetat extrahiert, die organische Phase mit Wasser gewaschen, über MgSO₄ getrocknet und zur Trockene eingedampft. Die Reinigung des Rohprodukts erfolgt durch Säulenchromatographie (Laufmittel: Toluol).
Ausbeute: 10,80 g Öl 1 (= 38 % d. Th.)

### I.2.: 2,2'-Dichlorbenzilsäuremethylester 3b:

Zu frisch hergestellter Natriumethanolatlösung aus 0,78 g (0,034 mol) Natrium und 100 ml Ethanol wird bei 20° C eine Lösung aus 10,0 g (0,034 mol) 2,2'-Dichlorbenzilsäure in 50 ml Ethanol zugetropft und über Nacht gerührt. Die Lösung wurde zur Trockene eingedampft, der Rückstand in DMF gelöst und bei 20° C tropfenweise mit 9,57 g (0,0674 mol) Methyljodid versetzt, 72 h nachgerührt.
In die entstandene Suspension wurden unter Eiskühlung 300 ml Wasser getropft, mit Diethylether extrahiert, die organische Phase mit Wasser gewaschen, über Na₂SO₄ getrocknet und zur Trockene eingedampft.
Ausbeute: 10,48 g 8 (= 100% d. Th.)

### I.3.: 4,4'-Difluorbenzilsäuremethylester 3c:.

### I.3.1.: 4,4'-Difluorbenzilsäure:

Zu einer Lösung aus 49,99 g (1,25 mol) NaOH-Plätzchen in 300 ml Wasser wird bei ca. 100° C eine Lösung aus 24,62 g (0,1 mol) 4,4'-Difluorbenzil in 250 ml Dioxan zugetropft und 2 h gerührt. Das Dioxan wird größtenteils abdestilliert und die verbleibende wässrige Lösung mit Dichlormethan extrahiert. Beim Ansäuern der wässrigen Lösung mit Schwefelsäure fällt ein Niederschlag aus, der abgesaugt, gewaschen und getrocknet wird. Das Filtrat wird mit Dichlormethan extrahiert, die organische Phase über Na₂SO₄ getrocknet und zur Trockene eingedampft.
Ausbeute: 25,01 g (= 95 % d. Th.); Smp.: 133°-136° C

### 1.3.2.: 4,4'-Difluorbenzilsäuremethylester:

Zu frisch hergestellter Natriumethanolatlösung aus 2,17 g (0,095 mol) Natrium und 200 ml Ethanol werden bei 20° C 25,0 g (0,095 mol) 4,4'-Difluorbenzilsäure zugegeben und 3 h gerührt. Die Lösung wird zur Trockene eingedampft, der Rückstand in DMF gelöst, bei 20° C tropfenweise mit 22,57 g (0,16 mol) Methyljodid versetzt und 24 h gerührt. Die Aufarbeitung und Reinigung erfolgt in Analogie zu Verbindung **3b.** Ausbeute: 21,06 g 11 (= 80 % d. Th.)

### I.4.: 2,2',4,4'-Tetrafluorbenzilsäuremethylester 3d:.

### I.4.1: 2,4-Difluorphenylglyoxylsäuremethylester:

50 g (0,44 mol) 1,3-Difluorbenzol werden bei 20° C in 135 ml Schwefelkohlenstoff gelöst, mit 73,5 g (0,55 mol) AlCl₃ und anschließend 55,15 g (0,45 mol) Oxalsäuremethylesterchlorid zugegeben und 2,5 h bei 20° C gerührt. Unter Kühlung wird eiskalte 2 N wässrige Salzsäure tropfenweise zugesetzt, mit Ethylacetat extrahiert, die organische Phase mit Wasser und 10 %iger wässriger Na₂CO₃-Lsg. gewaschen, über MgSO₄ getrocknet und zur Trockene eingedampft.
Ausbeute: 38,0 g Öl 14 (= 43 % d. Th.)

### I.4.2.: 2,2',4,4'-Tetrafluorbenzilsäuremethylester:

Ausgehend von dem gemäß vorstehender Vorschrift erhaltenen 2,4-Difluorphenylglyoxylsäuremethylester erfolgt die Herstellung der Titelverbindung analog zu 3a,
Ausbeute: 7,55 g 15 (= 13 % d. Th.).

### I.5.: 4.4'-Dimethylbenzilsäuremethylester 3e:.

Zu frisch hergestellter Natriumethanolatlösung aus 1,1 g (0,045 mol) Natrium und 100 ml Ethanol werden bei 20° C 12,43 g (0,048 mol) 4,4'-Dimethylbenzilsäure in 50 ml Ethanol zugetropft und 30 min. gerührt. Die Lösung wird zur Trockene eingedampft, der Rückstand in 50 ml DMF gelöst, bei 20° C tropfenweise 9,08 g (0,064 mol) Methyljodid zugegeben und weitere 24 h gerührt. In die entstandene Suspension werden unter Eiskühlung 300 ml Wasser getropft, mit Diethylether extrahiert, die organische Phase mit Wasser gewaschen, über Na₂SO₄ getrocknet und zur Trockene eingedampft. Ausbeute: 8,6 g (= 99 % d. Th.); Smp.: 83-84°C.

### I.6.: 3,3',4,4'-Tetramethoxybenzilsäuremethylester 3f:.

### I.6.1: 3,4-Dimethoxyglyoxylsäuremethylester.

14,00 g (0,11 mol) AlCl₃ werden in 100 ml Dichlormethan vorgelegt und bei 5°C mit 12,86 g (0,11 mol) Oxalsäuremonomethylesterchlorid versetzt. Zu der orangefarbenen Lösung werden bei 0° C 1,2-Dimethoxybenzol getropft, 1 h bei 0°C, dann 24 h bei 20° C nachgerührt, auf Eis/Salzsäure gegossen, extrahiert, die organische Phase mit Wasser und NAHCO₃-Lösung gewaschen, über MgSO₄ getrocknet und zur Trockene eingedampft. Der Rückstand wird aus Diethylether/Petrolether kristallisiert. Ausbeute: 13,55 g (= 60 % d. Th.);
Smp.: 65°-66° C.

### 1.6.2: 3,3',4,4'-Tetramethoxybenzilsäuremethylester:

Die Herstellung des Grignard-Reagenzes erfolgt aus 1,58g (0,065 mol) Magnesiumspäne, etwas Jod und 14,10 g (0,065 mol) Bromveratrol in 50 ml THF bei 50°C. Es wird 1 h nachgerührt. 11,28 g (0,05 mol) 3,4-Dimethoxyglyoxylsäuremethylester werden in 80 ml THF vorgelegt und bei 10°-15° C mit dem Grignard-Reagenz tropfenweise versetzt, 2 h bei 20° C gerührt, auf Eis/Schwefelsäure gegossen, mit Ethylacetat extrahiert. Anschließend wird die organische Phase mit Wasser gewaschen, über MgSO₄ getrocknet und zur Trockene eingedampft.
Die Reinigung erfolgt durch Kristallisation aus Aceton/Diethylether.
Ausbeute: 7,62 g (= 42 % d. Th.); Smp.: 129°-130° C.

### I.7.: 4,4'-Dimethoxybenzilsäuremethylester 3g:.

### I.7.1: 4-Methoxyglyoxylsäuremethylester:

Die Synthese erfolgt in Analogie zu Stufe I.6.1 ausgehend von 21.65 g (0,20 mol) Anisol; Ausbeute: 16,45 g (= 60 % d. Th.); Smp.: 52° C.

### I.7.2: 3,4-Dimethoxybenzilsäuremethylester:

Die Herstellung erfolgt in Analogie zu Stufe I.6.2, ausgehend von 16,45 g (0,085 mol) 4-Methoxyglyoxysäuremethylester; Die Reinigung erfolgt durch Kristallisation aus Isopropanol; Ausbeute: 12,28 g (= 48 % d. Th.); Smp.: 111 °C.

### I.8.: 3,3'-Dimethyl-4,4'-dimethoxybenzilsäuremethylester 3h:.

### I.8.1: 3-Methyl-4-methoxyglyoxylsäuremethylester:

Die Synthese erfolgt in Analogie zu Stufe 1.6.1 ausgehend von 26,88 g (0,22 mol) 2-Methylanisol; Ausbeute; 21,0 g (= 46 % d. Th.); Smp.: 49° C.

### I.8.2: 3,4-Dimethoxybenzilsäuremethylester:

Die Herstellung erfolgt in Analogie zu Stufe 1.6.2, ausgehend 21,0 g (0,1 mol) 3-Methyl-4-methoxyglyoxylsäuremethylester; Die Reinigung erfolgt durch Kristallisation aus Petrolether/Diethylether; Ausbeute: 11,1 g (= 33 % d. Th.); Smp.: 134°C.

### I.9.: 4,4'-Dichlorbenzilsäureethylester 3i:.

Die Synthese kann in Analogie zu Stufe I.5 erfolgen;

### I.10: 3,3',5,5'-Tetrafluorbenzilsäuremethylester 3i:.

### I.10.1: 3,3',5,5'-Tetrafluorbenzil:

110 ml Ethanol werden bei Raumtemperatur vorgelegt und 50,0 g (0,352 mol) 3,5-Difluorbenzaldehyd und 4,44 g (0,018 mol) 3-Ethyl-5-(2-hydroxyethyl)-4-methylthiazoliumbromid zugegeben. Anschließend werden 10,7 g (0,11 mol) Triethylamin zugetropft. Es wird 3 h unter Rückfluß gekocht und zur Trockene eingedampft. Der Rückstand wird in Ethylacetat aufgenommen und mit Wasser, Natriumpyrosulfit in Wasser und Na₂CO₃-Lösung extrahiert, Nach Trocknen über MgSO4 wird bis zur Trockene eingedampft.
Ausbeute: 39,91 g gelbe Kristalle (= 80% d. Th.).

39,91 g des so erhaltenen Acyloins werden in 1700 ml Acetonitril bei Raumtemperatur gelöst, 0,2 ml Vanadium-(V)-oxytrichlorid zugegeben und Sauerstoff eingeleitet. Nach 1,5 h wird die Lösung zur Trockene eingedampft, mit Ethylacetat und Wasser, sowie Na₂CO₃-Lösung extrahiert, über MgO₄ getrocknet und zur Trockene eingedampft, Der verbleibende Rückstand wird mit Petrolether/Ethylacetat 95:5 ausgerührt.
Ausbeute: 26,61 g gelbgrüne Kristalle (= 67% d. Th.); Smp.: 136-138°C.

### 1.10.2: 3,3',5,5'-Tetrafluorberizilsäure:

46,98 g (1,747 mol) Natriumhydroxid in 330 ml Wasser werden unter gutem Rühren im kochenden Wasserbad vorgelegt und eine Lösung aus 26,61 g (0,094 mol) 3,3',5,5'-Tetrafluorbenzil in 330 ml Dioxan zugetropft und anschließende 1 h nachgerührt. Nach Abkühlung wird das Dioxan eingedampft, der Rückstand mit Wasser verdünnt und mit Diethylether extrahiert. Die organische Phase wird sauer gestellt, mit Dichlormethan extrahiert, über MgSO4 getrocknet, zur Trockene eingedampft. Ausbeute: 20,15 g gelbliche Kristalle (= 71% d. Th.).

### 1.10.3: 3,3',5,5'-Tetrafluorbenzilsäuremethylester:

20,15 g (0,0671 mol) 3,3',5,5'-Tetrafluorbenzilsäure werden in 250 ml Acetonitril vorgelegt, 18,6 g (0,121 mol) DBU und 34,4 g (0,2426 mol) Methyljodid zugegeben und anschließend 6 h bei Raumtemperatur gerührt. Das Reaktionsgemisch wird zur Trockene eingedampft, der Rückstand mit Ethylacetat und Wasser extrahiert, die organische Phase über MgSO₄ getrocknet, zur Trockene eingedampft.
Die Umkristallisation erfolgt aus Cyclohexan.
Ausbeute: 15,11 g beige Kristalle (= 68% d. Th.); Smp.: 113-.114° C

### I.11: 3,3'-Dichlorbenzilsäuremethylester 3k:.

### I.11.1.: 3,3'-Dichlorbenzil:

100 ml Ethanol werden bei Raumtemperatur vorgelegt und 50,0 g (0,356 mol) 3-Chlorbenzaldehyd und 4,54 g (0,018 mol) 3-Ethyl-5-(2-hydroxyethyl)-4-methylthiazoliumbromid zugegeben. Anschließend werden 10,7 g (0,11 mol) Triethylamin zugetropft. Es wird 3 h unter Rückfluß gekocht und zur Trockene eingedampft. Der Rückstand wird in Ethylacetat aufgenommen und mit Wasser, Natriumpyrosulfit in Wasser und Na2CO3-Lösung extrahiert. Nach Trocknen über MgSO4 wird bis zur Trockene eingedampft. Das erhaltene Produkt wird aus Isopropanol und Petrolether umkristallisiert.
Ausbeute: 13,2 g weiße Kristalle (= 13% d. Th.); Smp.: 69-70°C.

13,0 g des so erhaltenen Acyloins werden in 460 ml Acetonitril bei RT gelöst, 0,0867 g Vanadium-(V)-oxytrichlorid zugegeben und Sauerstoff eingeleitet. Nach 1,5 h wird die Lösung zur Trockene eingedampft, mit Ethylacetat und Wasser, sowie Na2CO3-Lösung extrahiert, über MgSO4 getrocknet und zur Trockene eingedampft. Der verbleibende Rückstand wird mit Petrolether/Ethylacetat 95:5 ausgerührt. Ausbeute: 12,59 g gelbe Kristalle (= 97% d. Th.); Smp.: 116-117°C.

### I.11.2.: 3,3'-Dichlorbenzilsäure:

51,45 g (1,286 mol) Natriumhydroxid in 1000 ml Wasser werden unter gutem Rührern im kochenden Wasserbad vorgelegt und eine Lösung aus 28,5 g (0,102 mol) 3,3'-Dichlorbenzil in 700 ml Dioxan zugetropft und anschließende 1 h nachgerührt. Nach Abkühlung wird das Dioxan eingedampft, der Rückstand mit Wasser verdünnt und mit Diethylether extrahiert. Die organische Phase wird sauer gestellt, mit Dichlormethan extrahiert, über MgSO4 getrocknet, zur Trockene eingedampft. Ausbeute: 32,7 g (= 71 % d. Th.).

### I.11.3.: 3,3'-Dichlorbenzilsäuremethylester:

Aus 100 ml Ethanol und 1,97 g (0,0855 mol) Natrium wird eine Natriumethanolatlösung hergestellt, zu der 26,6 g (0,0855 mol) 3,3'-Dichlorbenzilsäure in 50 ml Ethanol getropft wird. anschließend wird 4 h bei Raumtemeperatur gerührt. Nach Abdestillieren des Lösungsmittels wird der Rückstand in 150 ml DMF gelöst und 24,27 g (0,171 mol) Methyljodid zugetropft, anschließend weitere 24 h gerührt. Unter Eiskühlung werden 300 ml Wasser und 200 ml Diethylether zugetropft, die Phasen getrennt, die Wasserphase mit Diethylether extrahiert, anschließend die organischen Phasen mit Na2CO3-Lösung gewaschen und mit Wasser neutral geschüttelt. Nach Trocknen über Na2SO4 wird zur Trockene eingedampft. Ausbeute: 22,91 g gelbes Öl (= 82% d. Th.).

### I.12: 4.4'-Di(trifluormethyl)benzilsäuremethylester 3I.

### I.12.1.: 4,4'-Di(trifluormethyl)benzil:

Die Umsetzung zum Acyloin-Derivat gelingt in Analogie zum Verfahren gemäß Stufe I.11.1 ausgehend von 174,1 g 4-(Trifluormethyl)-benzaldehyd.
Ausbeute: 150,2 g weiß-gelbe Kristalle (= 86% d. Th.).

150,2 g des so erhaltenen Acyloins werden in Analogie zur Durchführung gemäß Stufe 1.11.1 zum Benzil umgesetzt.
Ausbeute: 93,5 g gelbe Kristalle (= 63% d. Th.); Smp.: 141-142°C.

### I.12.2.: 4,4'-Di(trifluormethyl)benzilsäure:

10,00 g (0,0289 mol) 4,4'-Di(trifluormethyl)benzil werden analog zur Vorgehensweise nach Stufe I.11.2 zur entsprechenden Benzilsäure umgesetzt.
Ausbeute: 8,15 g gelbliche Kristalle (= 77% d. Th.).

### I.12.3,: 4,4'-Di(trifluormethyl)benzilsäuremethylester:

38,5 g (0,115 mol) 4,4'-Di(trifluormethyl)benzilsäure, 30,5 g (0,20 mol) DBU und 56,8 g (0,40 mol) Methyljodid werden in 400 ml Acetonitril analog zu Stufe 1.10.3 umgesetzt. Die Reinigung erfolgt mittels Flashchromatographie (Laufmittel Cyclohean/Ethylacetat 95:5). Ausbeute: 20,05 g weiße Kristalle (= 46% d. Th.) Smp.: 68° C

### I.13: 3,3'-Di(trifluormethyl)benzilsäuremethylester 3m:.

### I.13.1.: 3,3'-Di(trifluormethyl)benzil:

Die Umsetzung zum Acyloin-Derivat gelingt in Analogie zum Verfahren gemäß Stufe 1.11.1 ausgehend von 30,0 g 3-(Trifluormethyl)-benzaldehyd,
Ausbeute: 25,96 g gelbes Öl (= 87% d. Th.).

25,96 g des so erhaltenen Acyloins werden in Analogie zur Durchführung gemäß Stufe I.11.1 zum Benzil umgesetzt.
Ausbeute: 10,5 g hellgelbe Kristalle (= 24% d. Th.).

### 1.13.2.: 3,3'-Di(trifluormethyl)benzilsäure:

10,5 g (0,0182 mol) 3,3'-Di(trifluormethyl)benzil werden analog zur Vorgehensweise nach Stufe I.11.2 zur entsprechenden Benzilsäure umgesetzt.
Ausbeute: 10,55 g gelbliches Öl.

### I.13.3.: 3,3'-Di(trifluormethyl)benzilsäuremethylester;

10,55 g (0,0289 mol) 4,4'-Di(trifluormethyl)benzilsäure, 8,82 g (0,0579 mol) DBU und 16,44 g (0,1158 mol) Methyljodid werden in 110 ml Acetonitril analog zu Stufe 1.10.3 umgesetzt. Die Reinigung erfolgt mittels Umkristallisation aus Cyclohexan. Ausbeute: 6,02 g weiße Kristalle (= 57% d. Th.); Smp.: 69-70°C.

### I.14: 3,3'-Dichlor-4,4'-difluorbenzilsäuremethylester 3n:.

### I.14.1.: 3,3'-Dichlor-4,4'-difluorbenzil:

Die Umsetzung zum Acyloin-Derivat gelingt in Analogie zum Verfahren gemäß Stufe I.11.1 ausgehend von 30,0 g 3-Chlor-4-fluor-benzaldehyd.
Ausbeute: 29,49 g orangenes Öl,

29,49 g des so erhaltenen Acyloins werden in Analogie zur Durchführung gemäß Stufe I.11.1 zum Benzil umgesetzt.
Ausbeute: 24,88 g gelbe Kristalle.

### I.14.2.: 3,3'-Dichlor-4,4'-difluorbenzilsäure:

24,88 g (0,079 mol) 3,3'-Dichlor-4,4'-difluorbenzil werden analog zur Vorgehensweise nach Stufe I.11,2 zur entsprechenden Benzilsäure umgesetzt. Ausbeute: 17,07g orangener Feststoff.

### 1.14.3.: 3,3'-Dichlor-4,4'-difluorbenzilsäuremethylester:

17,07 g (0,0512 mol) 3,3'-Dichlor-4,4'-difluorbenzilsäure, 14,10 g (0,0926 mol) DBU und 26,29 g (0,1852 mol) Methyljodid werden in 200 ml Acetonitril analog zu Stufe I.10.3 umgesetzt, Ausbeute: 6,77 g (= 38% d. Th.).

### I.15:_2,2',5,5'-Tetrafluorbenzilsäuremethylester 3o:.

### I.15.1.: 2,2',5,5'-Tetrafluorbenzil:

Die Umsetzung zum Acyloin-Derivat gelingt in Analogie zum Verfahren gemäß Stufe I.11.1 ausgehend von 50,0 g 2,5-Difluor-benzaldehyd,
Ausbeute: 45,5 g gelbe Kristalle.

45,5 g des so erhaltenen Acyloins werden in Analogie zur Durchführung gemäß Stufe I.11.1 zum Benzil umgesetzt.
Ausbeute: 39,75 g gelbe Kristalle (=88% d. Th.),

### I.15.2.: 2,2',5,5'-Tetrafluorbenzilsäure:

39,75 g (0,14 mol) 2,2',5,5'-Tetrafluorbenzil werden analog zur Vorgehensweise nach Stufe 1.11.2 zur entsprechenden Benzilsäure umgesetzt.
Ausbeute: 44, 76 g gelbliches Öl.

### 1.15.3.: 2,2',5,5'-Tetrafluorbenzilsäuremethylester:

44,76 g (0,14 mol) 2,2',5,5'-Tetrafluorbenzilbenzilsäure werden analog zu Stufe I.10.3 umgesetzt. Ausbeute: 29,4 g Öl.

### I.16: 2,2',3,3'-Tetrafluorbenzilsäuremethylester 3p:.

### I.16,1.: 2,2',3,3'-Tetrafluorbenzil:

Die Umsetzung zum Acyloin-Derivat gelingt in Analogie zum Verfahren gemäß Stufe I.11.1 ausgehend von 30,0 g 2,3-Difluor-benzaldehyd.
Ausbeute: 29,85 g gelbe Kristalle.

29,85 g des so erhaltenen Acyloins werden in Analogie zur Durchführung gemäß Stufe I.11.1 zum Benzil umgesetzt.
Ausbeute: 25,22 g orangenes Öl.

### I.16.2.: 2,2',3,3'-Tetrafluorbenzilsäure:

25,22 g (0,0894 mol) 2,2',3,3'-Tetrafluorbenzil werden analog zur Vorgehensweise nach Stufe I.11.2 zur entsprechenden Benzilsäure umgesetzt.
Ausbeute: 29, 13 g orangener Feststoff.

### I.16.3.; 2,2',3,3'-Tetrafluorbenzilsäuremethylester:

29,13 g (0,097 mol) 2,2',3,3'-Tetrafluorbenzilbenzilsäure werden analog zu Stufe I.10.3 umgesetzt. Ausbeute: 15,78 g beige Kristalle; Smp.: 102-103°C.

### I.17: 3,3'-Difluorbenzilsäuremethylester 3g:.

### I.17.1.: 3,3'-Difluorbenzil:

Die Umsetzung zum Acyloin-Derivat gelingt in Analogie zum Verfahren gemäß Stufe I.11.1 ausgehend von 50,0 g 3-Fluor-benzaldehyd.
Ausbeute: 49,45 g.

49,45 g des so erhaltenen Acyloins werden in Analogie zur Durchführung gemäß Stufe I.11.1 zum Benzil umgesetzt.
Ausbeute: 42,01 g hellgelbe Kristalle.; Smp.: 104-105°C.

### I.17.2.: 3,3'-Difluorbenzilsäure;

42,01 g (0,171 mol) 3,3'-Difluorbenzil werden analog zur Vorgehensweise nach Stufe I.11.2 zur entsprechenden Benzilsäure umgesetzt.
Ausbeute: 33,07 g (=75% d. Th.); Smp.: 121-122°C.

### I.17.3.: 3,3'-Difluorbenzilsäuremethylester:

33,7 g (0,128 mol) 3,3'-Difluorbenzilbenzilsäure werden analog zu Stufe 1.10.3 umgesetzt. Ausbeute: 34,78 g beige Kristalle (=98% d. Th.); Smp.: 84-85°C.

### I.18: 4,4'-Dichlor-3,3'-difluorbenzilsäuremethylester 3r:.

### I.18.1.: 4,4'-Dichlor-3,3'-difluorbenzil:

Die Umsetzung zum Acyloin-Derivat gelingt in Analogie zum Verfahren gemäß Stufe I.11.1 ausgehend von 33,13 g 4-Chlor-3-fluor-benzaldehyd.
Ausbeute: 30,07 g Öl.

30,07 g des so erhaltenen Acyloins werden in Analogie zur Durchführung gemäß Stufe I.11.1 zum Benzil umgesetzt.
Ausbeute: 19,32 g hellgelbes Pulver.

### I.18.2.: 4,4'-Dichlor-3,3'-difluorbenzilsäure:

19,32 g (0,0613 mol) 4,4'-Dichlor-3,3'-difluorbenzil werden analog zur Vorgehensweise nach Stufe I.11.2 zur entsprechenden Benzilsäure umgesetzt. Ausbeute: 25,3 g Öl.

### I.18.3.: 4,4'-Dichlor-3,3'-difluorbenzilsäuremethylester:

25,3 g (0,075 mol) 4,4'-Dichlor-3,3'-difluorbenzilsäure werden analog zu Stufe I.10.3 umgesetzt. Ausbeute: 13,07 g gelbes Öl (= 50% d. Th.).

### I.19: 3,3',4,4'-Tetrachlorbenzilsäuremethylester 3s:.

### I.19.1.: 3,3',4,4'-Tetrachlorbenzil:

Die Umsetzung zum Acyloin-Derivat gelingt in Analogie zum Verfahren gemäß Stufe I.11.1 ausgehend von 100 g 3,4-Dichlorbenzaldehyd.
Ausbeute: 60,89 g Öl.

60,89 g des so erhaltenen Acyloins werden in Analogie zur Durchführung gemäß Stufe I.11.1 zum Benzil umgesetzt.
Ausbeute: 42,45 g gelbe Kristalle.
I.19.2.: 3,3',4,4'-Tetrachlorbenzilsäure:

44,75 g (0,128 mol) 3,3',4,4'-Tetrachlorbenzil werden analog zur Vorgehensweise nach Stufe I.11.2 zur entsprechenden Benzilsäure umgesetzt.
Ausbeute: 42 g gelbliches Pulver, Smp.: 224°C.

### I.19.3.: 3,3',4,4'-Tetrachlorbenzilsäuremethylester:

42 g (0,114 mol) 3,3',4,4'-Tetrachlorbenzilsäure werden analog zu Stufe I.10.3 umgesetzt. Ausbeute: 15,84 g (= 37% d. Th.); Smp.: 69°C.

### I.20: 3,3',4,4',5,5'-Hexafluorbenzilsäuremethylester 3t:.

### I.20.1.: 3,3',4,4',5,5'-Hexafluorbenzil:

Die Umsetzung zum Acyloin-Derivat gelingt in Analogie zum Verfahren gemäß Stufe I.11.1 ausgehend von 31,38 g 3,4,5-Trifluorbenzaldehyd.
Ausbeute: 24,92 g.

24,92 g des so erhaltenen Acyloins werden in Analogie zur Durchführung gemäß Stufe I.11.1 zum Benzil umgesetzt.
Ausbeute: 14,65 g gelbe Kristalle.

### I.20.2.: 3,3',4,4',5,5'-Hexafluorbenzilsäure:

14,62 g (0,046 mol) 3,3',4,4',5,5'-Hexafluorbenzil werden analog zur Vorgehensweise nach Stufe I.11.2 zur entsprechenden Benzilsäure umgesetzt.
Ausbeute: 15,77 g gelbe Kristalle.

### I.20.3.: 3,3',4,4',5,5'-Hexafluorbenzilbenzilsäuremethylester:

42 g (0,114 mol) 3,3',4,4'-Tetrachlorbenzilsäure werden analog zu Stufe I.10.3 umgesetzt. Ausbeute: 5,65 g (= 35% d. Th.); Smp.: 82-83°C.

### II. Synthese der Verbindungen der allgemeinen Formen 4:

### II.1.: 3,3',4,4'-Tetrafluorbenzilsäuretropenolester 4.1:

4,27 g (0,013 mol) 3,3',4,4'-Tetrafluorbenzilsäureethylester **3a,** 1,81 g (0,013 mol) Tropenol und 0,03 g Natrium werden als Schmelze bei 75 mbar 4 h auf kochendem Wasserbad unter gelegentlichem Schütteln erhitzt. Nach Abkühlung werden die Natriumreste mit Acetonitril aufgelöst, die Lösung zur Trockene eingedampft und der Rückstand mit Dichlormethan/Wasser extrahiert. Die organische Phase wird mit Wasser gewaschen, über MgSO₄ getrocknet und zur Trockene eingedampft. Der verbleibende Rückstand wird mit Diethylether/Petrolether 1:9 versetzt, abgesaugt und gewaschen. Ausbeute: 2,50 g (= 46 % d. Th.);
DC: Rf-Wert: 0,29 (Laufmittel: sek. Butanol/Ameisensäure/Wasser 75:15:10);
Smp.: 147°-148°C.

### II.2.: 3,3',4,4'-Tetrafluorbenzilsäurescopinester 4.2:

Die Darstellung von 4.2 erfolgt in Analogie zum Verfahren nach II.1;
Ausbeute: 1,75 g (= 36 % d. Th.) ausgehend von 3,61 g (0,011 mol) 3a und 1,71 g (0.011 mol) Scopin; DC: Rf-Wert: 0,22 (Laufmittel analog Stufe II.1);
Smp.: 178-179°C;

### II.3.: 4,4'-Dichlorbenzilsäuretropenolester 4.3:

Die Darstellung von **4.3** erfolgt in Analogie zum Verfahren nach II.1;
Ausbeute: 6,95 g (= 83 % d. Th.) ausgehend von 6,5 g (0,02 mol) **3i** und 2,78 g (0.02 mol) Tropenol; DC: Rf-Wert: 0,30 (Laufmittel analog Stufe II.1); Smp.: 197-199°C;

### II.4.: 2,2'-Dichlorbenzilsäuretropenolester 4.4:

Die Darstellung von **4.4** erfolgt in Analogie zum Verfahren nach II.1; das Produkt wurde als Hydrochlorid gefällt und aus Acetonitril umkristallisiert;
Ausbeute: 1,13 g (= 8 % d. Th.) ausgehend von 9,3 g (0,03 mol) **3b** und 8,32 g (0.06 mol) Tropenol; DC: Rf-Wert: 0,26 (Laufmittel analog Stufe II. 1); Smp.: 253-256°C (Hydrochlorid);

### II.5.: 4,4'-Difluorbenzilsäuretropenolester 4.5:

Die Darstellung von **4.5** erfolgt in Analogie zum Verfahren nach II.1;
Ausbeute: 8,71 g (= 69 % d. Th.) ausgehend von 8,35 g (0,03 mol) **3c** und 4,18 g (0.03 mol) Tropenol; DC: Rf-Wert: 0,34 (Laufmittel analog Stufe II.1);
Smp.: 167-169°C;

### II.6.: 2,2',4,4'-Tetrafluorbenzilsäuretropenolester 4.6:

Die Darstellung von **4.6** erfolgt in Analogie zum Verfahren nach III.1;
Ausbeute: 1,80 g (= 27 % d. Th.) ausgehend von 4,00 g (0,013 mol) **3d** und 3,54 g (0.036 mol) Tropenol; Smp.: 190°C;

### II.7.: 4.4'-Dichlorbenzilsäurescopinester 4.7:

3,78 g (0,01 mol) 4,4'-Dichlorbenzilsäuretropenolester **4.3** werden in 40 ml DMF suspendiert, auf 60°C erhitzt bis eine klare Lösung entsteht. Bei ca. 40°C Innentemperatur wird eine Lösung aus 1,92 g (0,0216 mol) H₂O₂-Harnstoff in 10 ml Wasser, sowie 0,183 g (0,0011 mol) Vanadium-(V)-oxid zugegeben und 4,5 h bei 60°C gerührt. Nach Abkühlen auf 20°C wird der entstandene Niederschlag abgesaugt, das Filtrat mit 4 N Salzsäure auf pH 3 gestellt, mit 0,437 g (0,0023 mol) Na₂S₂O₇ in 10 ml Wasser versetzt. Die dadurch entstandene grüne Lösung wird zur Trockene eingedampft, der Rückstand mit Dichlormethan/Wasser extrahiert. Die saure Wasserphase wird mit Na₂CO₃ basisch gestellt, mit Dichlormethan extrahiert und die organische Phase über Na₂SO₄ getrocknet und konzentriert. Anschließend erfolgte die Zugabe von 0,5 ml Acetylchlorid bei ca. 15°C und 1,5 h Rühren. Nach Extraktion mit 0,1 N Salzsäure wird die Wasserphase basisch gestellt, mit Dichlormethan extahiert, die organische Phase über Na₂SO₄ getrocknet und zur Trockene eingedampft. Aus dem Rückstand wird das Hydrochlorid gefällt und aus Methanol/Diethylether umkristallisiert.
Ausbeute: 1,92 g 18 (= 45 % d. Th.); DC: Rf-Wert: 0,29 (Laufmittel wie in II.1);
Smp.: 238-239°C (Hydrochlorid).

### II.8.: 4,4'-Difluorbenzilsäurescopinester 4.8:

Die Darstellung von **4.8** erfolgt in Analogie zum Verfahren nach II.7;
Ausbeute: 2,60 g (= 70 % d. Th.) ausgehend von 3,27 g (0,09 mol) **4.5**;
DC: Rf-Wert: 0,25 (Laufmittel analog Stufe II.1); Smp.: 243-244°C (Hydrochlorid).

### II.9.: 4,4'-Dibrombenzilsäuretropenolester 4.9:

Die Darstellung von **4.9** erfolgt in Analogie zum Verfahren nach II.1;
Ausbeute: 2,36 g (= 19 % d. Th.) ausgehend von 9,9 g (0,023 mol) käuflich erhältlichem 4,4'-Dibrombenzilsäureisopropylester und 3,21 g (0.023 mol) Tropenol; zur Reinigung wurde das Hydrochlorid gefällt und dieses aus Acetonitril umkristallisiert; DC: Rf-Wert: 0,30 (Laufmittel analog Stufe II.1);
Smp.: 205-207°C (Hydrochlorid).

### II.10.: 4,4'-Dimethylbenzilsäuretropenolester 4.10:

Die Darstellung von **4.10** erfolgt in Analogie zum Verfahren nach II.1;
Ausbeute: 3,55 g (= 81 % d. Th.) ausgehend von 2,87 g (0,01 mol) **3e** und 1,48 g (0.01 mol) Tropenol; zur Reinigung wurde das Hydrochlorid gefällt und dieses aus Acetonitril umkristallisiert; Smp.: 232-233°C (Hydrochlorid).

### II.11.: 4,4'-Dimethylbenzilsäurescopinester 4.11:

Die Darstellung von **4.11** erfolgt in Analogie zum Verfahren nach II.1;
Ausbeute: 1,02 g (= 24 % d. Th.) ausgehend von 2,87 g (0,01 mol) **3e** und 1,65 g (0.01 mol) Scopin; zur Reinigung wurde das Hydrochlorid gefällt;
Smp.: 181-183°C (Hydrochlorid).

### II.12.: 3,3'4,4'-Tetrafluorbenzilsäuretropinester 4.12:

Die Darstellung von **4.12** erfolgt in Analogie zum Verfahren nach II.1;
Ausbeute: 2,35 g (= 53 % d. Th.) ausgehend von 3,45 g (0,01 mol) **3a** und 1,49 g (0.01 mol) Tropin; Smp.: 142-144°C.

### II.13.: 3,3',4,4'-Tetramethoxybenzilsäuretropenolester 4.13:

2,60 g (0,007 mol) **3f**, 1,00 g (0,007 mol) Tropenol und 0,03 g Natrium werden in 15 ml Toluol vorgelegt und 4 h unter Rückfluß gekocht. Nach Abkühlen wird mit ca. 100 ml Toluol verdünnt, mit Wasser extrahiert, die organische Phase über MgSO₄ getrocknet und zur Trockene eingedampft. Ausbeute: 1,60 g Öl (= 47 % d. Th.)

### II.14.: 4,4'-Dimethoxybenzilsäuretropenolester 4.14:

Die Darstellung von **4.14** erfolgt in Analogie zum Verfahren nach II.1;
Ausbeute: 3,3 g (= 78 % d. Th.) ausgehend von 3,0 g (0,01 mol) **2g** und 1,39 g (0,01 mol) Tropenol, Smp.: 146-147°C.

### II.15.: 3,3'4,4'-Tetramethoxybenzilsäuretropinester 4.15:

Die Darstellung von **4.15** erfolgt in Analogie zum Verfahren nach II.1;
Ausbeute: 1,65 g (= 32 % d. Th.; Öl) ausgehend von 4,0 g (0,02 mol) 3f und 3,12 g (4.02 mol) Tropin.

### II.16.: 3,3',4,4'-Tetramethoxybenzilsäurescopinester 4.16:

Die Darstellung von **4.16** erfolgt in Analogie zum Verfahren nach II.7;
Ausbeute: 0,8 g (= 41 % d. Th., Öl) ausgehend von 1,857 g (0,004 mol) **4.13.**

### II.17.: 3,3'-Dimethyl-4,4'-Dimethoxybenzilsäuretropenolester 4.17:

Die Darstellung von **4.17** erfolgt in Analogie zum Verfahren nach II.1; die Reinigung erfolgt durch Umkristallisieren aus Diethylether; Ausbeute: 2,30 g (= 35 % d. Th.) ausgehend von 5,0 g (0,015 mol) **3h** und A,21 g (0.03 mol) Tropenol; Smp.: 126°C.

### II.18.: 3,3'-Dimethyl-4,4'-Dimethoxybenzilsäurescopinester 4.18;

Die Darstellung von **4.18** erfolgt in Analogie zum Verfahren nach II.7;
Ausbeute: 0,6 g (= 44 % d. Th., Öl) ausgehend von 1,3 g (0,003 mol) **4.17**.

### II.19.: 3,3',5,5'-Tetrafluorbenzilsäuretropenolester 4.19:

1,53 g (0,0636 mol) NaH werden in 30 ml Toluol vorgelegt, eine Lösung aus 14 g (0,0446 mol) 3j und 8,85 g (0,0636 mol) Tropenol in 80 ml Toluol wird bei 10°C/860 mbar zugetropft. Der bei der Reaktion entstehende Alkohol wird unter gleichzeitigem Zutropfen von Toluol abdestilliert. Nach 3 h wird abgekühlt, mit Dichlormethan und Wasser extrahiert. Die vereinigten organischen Phasen wurden über MgSO₄ getrocknet und zur Trockene eingedampft. Der Rückstand mit Petrolether/Ethylacetat 95:5 ausgerührt.
Ausbeute: 11,21 g hellbeige Kristalle (= 60% d. Th.); Smp.: 168°-170° C.

### II.20.: 2,2',4,4',-Tetrafluorbenzilsäurescopinester 4.20:

Die Darstellung von **4.20** erfolgt in Analogie zum Verfahren nach II.7;
Ausbeute: 1,05 g weiße Kristalle (= 89 % d. Th.) ausgehend von 1,15 g (0,0027 mol) **4.6.**

### II.21.: 3,3',5,5'-Tetrafluorbenzilsäurescopinester 4.21:

Die Darstellung von **4.21** erfolgt in Analogie zum Verfahren nach II.7;
Ausbeute: 1,13 g weiße Kristalle (= 55 % d. Th.) ausgehend von 2,0 g (0,0047 mol) **4.19.** Smp.: 199-200°C.

### II.22.: 3,3'-Dichlorbenzilsäuretropenolester 4.22:

22,9 g (0,074 mol) 3,3'-Dichlorbenzilsäuremethylester **3k**, 15,37 g (0,11 mol) Tropenol und 0,17 g Natrium werden als Schmelze bei 75 mbar 4 h auf kochendem Wasserbad unter gelegentlichem Schütteln erhitzt. Nach Abkühlung werden die Natriumreste mit Acetonitril aufgelöst, die Lösung zur Trockene eingedampft und der Rückstand mit Dichlormethan/Wasser extrahiert. Die organische Phase wird mit Wasser gewaschen, über MgSO₄ getrocknet und zur Trockene eingedampft.
Das Produkt wird in Form seines HYdrochlorids aus Acetonitril umkristallisiert. Ausbeute: 16,83 g weiße Kristalle (= 50 % d. Th.);
Smp.: 184-185°C.

### II.23.: 4,4'-Di(trifluormethyl)benzilsäuretropenolester 4.23:

Ausgehend von 10,0 g (0,0264 mol) **3l** wird die Reaktion in Analogie zu Stufe II.1 durchgeführt. Ausbeute: 4,70 g beige Kristalle (= 37 % d. Th.);
Smp.: 155°C.

### II.24.: 3,3'-Di(trifluormethyl)benzilsäuretropenolester 4.24:

Ausgehend von 6,01 g (0,0159 mol) **3m** wird die Reaktion in Analogie zu Stufe II.1 durchgeführt. Ausbeute: 3,03 g weiße Kristalle (= 39 % d. Th.);
Smp.; 124-125°C.

### II.25.: 4,4'-Di(trifluormethyl)benzilsäurescopinester 4.25:

Die Darstellung von **4.25** erfolgt in Analogie zum Verfahren nach II.7;
Ausbeute: 0,95 g klares Öl (= 46 % d. Th.) ausgehend von 2,0 g (0,0041 mol) **4.23.**

### II.26.: 3,3'-Di(trifluormethyl)benzilsäurescopinester 4.26:

Die Darstellung von **4.26** erfolgt in Analogie zum Verfahren nach II.7;
Ausbeute: 1,02 g weiße Kristalle (= 51 % d. Th.) ausgehend von 1,94 g (0,0039 mol) **4.24.**

### II.27.: 3,3'-Dichlor-4,4'-difluorbenzilsäuretropenolester 4.27:

Ausgehend von 6,77 g (0,0195 mol) **3n** wird die Reaktion in Analogie zu Stufe II.1 durchgeführt. Das erhaltene Rohprodukt wird aus Acetonitril umkristallisiert.
Ausbeute: 4,05 g hellbeige Kristalle (= 46 % d. Th.);
Smp.: 177-179°C.

### II.28.: 3,3'-Dichlorbenzilsäurescopinester 4.28 :

Die Darstellung von **4.28** erfolgt in Analogie zum Verfahren nach II.7;
Ausbeute: 7,86 g (= 75 % d. Th.) ausgehend von 10,37 g (0,024 mol) **4.22.**
Smp.: 174-175°C.

### II.29.: 2,2',5,5'-Tetrafluorbenzilsäuretropenolester 4.29:

Ausgehend von 6,29 g (0,02 mol) **3o** wird die Reaktion in Analogie zu Stufe II.1 durchgeführt. Das erhaltene Rohprodukt wird als Hydrochlorid aus Aceton umkristallisiert. Ausbeute: 0,89 g weiße Kristalle (= 10 % d. Th.);
Smp.: 177-179°C.

### II.30.: 2,2',3,3'-Tetrafluorbenzilsäuretropenolester 4.30:

Ausgehend von 8,0 g (0,0255 mol) **3p** wird die Reaktion in Analogie zu Stufe II.1 durchgeführt. Das erhaltene Rohprodukt wird aus Acetonitril umkristallisiert.
Ausbeute: 0,96 g beige Kristalle (= 9 % d. Th.);
Smp.: 176-177°C.

### II.31.: 3,3'-Difluorbenzilsäuretropenolester 4.31:

Ausgehend von 11,13 g (0,04 mol) **3a** wird die Reaktion in Analogie zu Stufe II.1 durchgeführt. Das erhaltene Rohprodukt wird als Hydrochlorid aus Acetonitril umkristallisiert. Ausbeute: 7,98 g (= 47 % d. Th.); Smp.: 245-246°C.

### II.32.: 3,3'-Difluorbenzilsäurescopinester 4.32:

Die Darstellung von **4.32** erfolgt in Analogie zum Verfahren nach II.7;
Ausbeute: 4,2 g (= 76 % d. Th.) ausgehend von 4,89 g (0,013 mol) **4.31.**
Smp.: 216-218°C.

### II.33.: 3,3'-Dichlor-4,4'-difluorbenzilsäurescopinester 4.33:

Die Darstellung von **4.33** erfolgt in Analogie zum Verfahren nach II.7;
Ausbeute: 0,78 g weiße Kristalle (= 34 % d. Th.) ausgehend von 4,6 g (0,0098 mol) **4.27**; Smp.: 216-218°C.

### II.34.: 4.4'-Dichlor-3,3'-difluorbenzilsäuretropenolester 4.34:

Ausgehend von 12,0 g (0,0345 mol) **3r** wird die Reaktion in Analogie zu Stufe II.1 durchgeführt. Das erhaltene Rohprodukt wird aus Petrolether ausgerührt.
Ausbeute: 6,65 g cremeweißes Pulver (= 42 % d. Th.);
Smp.: 180-181°C.

### II.35.: 4,4'-Dichlor-3,3'-difluorbenzilsäurescopinester 4.35:

Die Darstellung von **4.35** erfolgt in Analogie zum Verfahren nach II.7;
Ausbeute: 2,58 g weiße Kristalle (= 62 % d. Th.) ausgehend von 4,0 g (0,0088 mol) **4.34;** Smp.: 150-151°C.

### II.36.: 3,3',4,4'-Tetrachlorbenzilsäuretropenolester 4.36:

Ausgehend von 14,24 g (0,0375 mol) **3s** wird die Reaktion in Analogie zu Stufe II.1 durchgeführt. Das erhaltene Rohprodukt wird aus Acetonitril umkristallisiert.
Ausbeute: 4,81 g weiße Kristalle (= 26 % d. Th.);
Smp.: 149-150°C.

### II.37.: 3,3',4,4',5,5'-Hexafluorbenzilsäuretropenolester 4.37:

Ausgehend von 5,0 g (0,0143 mol) **3t** wird die Reaktion in Analogie zu Stufe II.7 durchgeführt. Das erhaltene Rohprodukt wird aus Acetonitril umkristallisiert.
Ausbeute: 3,49 g weiße Kristalle (= 53 % d. Th.);
Smp.: 164-165°C.

### III. Synthese der Verbindungen der allgemeinen Formel 1:

### Beispiel 1: 3,3',4,4'-Tetrafluorbenzilsäuretropenolester-Methobromid :

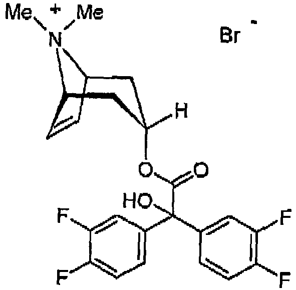

2,00 g (0,0048 mol) **4.1**, 30 ml Acetonitril, 10 ml Dichlormethan und 2,88 g (0,0143 mol) 46,92 % iges Brommethan in Acetonitril werden bei 20°C zusammengegeben und 3 Tage stehen gelassen. Die Lösung wird zur Trockene eingedampft und der Rückstand aus Acetonitril umkristallisiert. Ausbeute: 1,95 g (= 80 % d. Th.)
DC: Rf-Wert: 0,12 (Laufmittel analog Stufe II.1); Smp.: 238°C.
C₂₃H₂₂F₄NO₃xBr (516,33);

| | |
|---|---|
| Elementaranalyse: | berechnet: C (53,50) H (4,29) N (2,71) |
| | gefunden.: C (53,52) H (4,30) N (2,65). |

### Beispiel 2: 3,3',4,4'-Tetrafluorbenzilsäurescopinester-Methobromid :

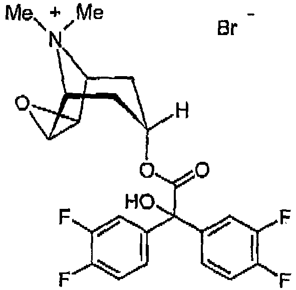

1,5 g (0,0034 mol) **4.2**,20 ml Acetonitril, 20 ml Dichlormethan und 2,08 g (0,01 mol) 46,92 % iges Brommethan in Acetonitril werden analog zu Beispiel 1 umgesetzt.
Ausbeute: 1,40 g (= 77 % d. Th.)
DC: Rf-Wert: 0,16 (Laufmittel analog Stufe II.1); Smp.: 227°C.
C₂₃H₂₂F₄NO₄xBr (532,33);

| | |
|---|---|
| Elementaranalyse: | berechnet: C (51,90) H (4,17) N (2,63) |
| | gefunden: C (51,91) H (4,16) N (2,60). |

### Beispiel 3: 4,4'-Dichlorbenzilsäuretropenolester-Methobromid (nicht gemäß der Erfindung):

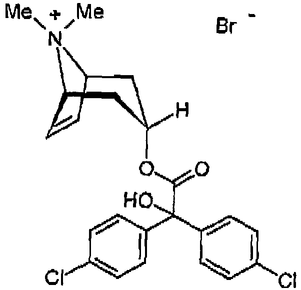

2,09 g (0,005 mol) **4.3** werden analog zu Beispiel 1 umgesetzt. Die entstandenen Kristalle werden abesaugt, mit Dichlormethan gewaschen, getrocknet und anschließend aus Methanol/Diethylether umkristallisiert.
Ausbeute: 1,72 g (=67 % d. Th.)
C₂₃H₂₄Cl₂NO₃xBr (513,26);
DC: Rf-Wert: 0,12 (Laufmittel analog Stufe II.1); Smp.: 195-196°C.

| | | | | |
|---|---|---|---|---|
| Elementaranalyse: | berechnet: | C (53,82) | H (4,71) | N (2,73) |
| | gefunden.: | C (53,54) | H (4,80) | N (2,73). |

### Beispiel 4: 2,2'-Dichlorbenzilsäuretropenolester-Methobromid (nicht gemäß der Erfindung):

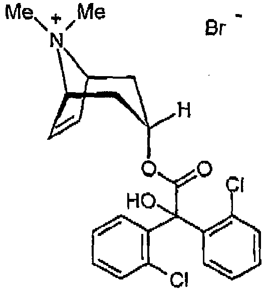

0,86 g (0,0021 mol) der freien Base von **4.4** werden analog zu Beispiel 1 umgesetzt. Die entstandenen Kristalle werden abesaugt, mit Aceton gewaschen, getrocknet und anschließend aus Methanol/Diethylether umkristallisiert.
Ausbeute: 0,99 g (=94 % d. Th.)
DC: Rf-Wert: 0,14 (Laufmittel analog Stufe II.1); Smp.: 260-261°C.
C₂₃H₂₄Cl₂NO₃xBr (513,26);

| | | | | |
|---|---|---|---|---|
| Elementaranalyse: | berechnet: | C (53,82) | H (4,71) | N (2,73) |
| | gefunden.: | C (53,62) | H (4,76) | N (2,69). |

### Beispiel 5: 4,4'-Difluorbenzilsäuretropenolester-Methobromid :

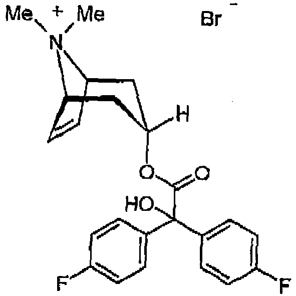

1,9 g (0,005 mol) der freien Base von **4.5** werden analog zu Beispiel 1 umgesetzt.
Ausbeute: 2,1 g (=89 % d. Th.)
DC: Rf-Wert: 0,14 (Laufmittel analog Stufe II,1); Smp.: 219-220°C.
C₂₃H₂₄F₂NO₃xBr (480,35);

| | | | | |
|---|---|---|---|---|
| Elementaranalyse: | berechnet: | C (57,51) | H (5,04) | N (2,92) |
| | gefunden.: | C (57,33) | H (4,86) | N (2,90). |

### Beispiel 6:2,2',4,4'-Tetrafluorbenzilsäuretropenolester-Methobromid :

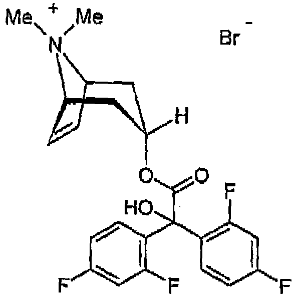

1,60 g (0,004 mol) **4.6** werden analog zu Beispiel 1 umgesetzt. Die entstandenen Kristalle werden abesaugt und aus Aceton umkristallisiert.
Ausbeute: 1,70 g (=87 % d. Th.)
DC: Rf-Wert: 0,13 (Laufmittel: n-Butanol/Wasser/Ameisensäure(konz.)/Aceton/Dichlormethan 36:15:15:15:5); Smp.: 241-242°C.
C₂₃H₂₂F₄NO₃xBr (516,33);

| | | | | |
|---|---|---|---|---|
| Elementaranalyse: | berechnet: | C (53,50) | H (4,29) | N (2,71) |
| | gefunden.: | C (53,55) | H (4,33) | N (2,73), |

### Beispiel 7: 4,4'-Dichlorbenzilsäurescopinester-Methobromid (nicht gemäß der Erfindung) :

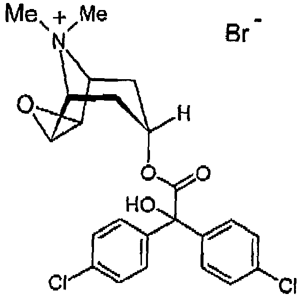

1,54 g (0,0035 mol) der freien Base von **4.7** werden analog zu Beispiel 1 umgesetzt. Die entstandenen Kristalle werden abesaugt, mit Aceton gewaschen, getrocknet und anschließend aus Methanol/Diethylether umkristallisiert.
Ausbeute: 1,68 g (=90 % d. Th.)
DC: Rf-Wert: 0,22 (Laufmittel analog Stufe II.1); Smp.: 209-210°C.
C₂₃H₂₄Cl₂NO₄xBr (529,26);

| | | | | |
|---|---|---|---|---|
| Elementaranalyse: | berechnet: | C (52,20) | H (4,57) | N (2,65) |
| | gefunden.: | C (51,25) | H (4,83) | N (2,49). |

### Beispiel 8: 4,4'-Difluorbenzilsäurescopinester-Methobromid :

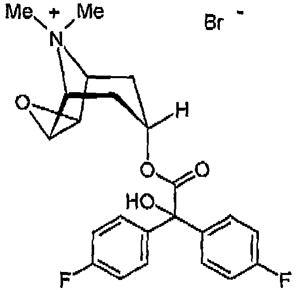

1,50 g (0,003 mol) der freien Base von **4.8** werden analog zu Beispiel 1 umgesetzt. Die entstandenen Kristalte werden abesaugt, mit Aceton gewaschen, getrocknet und anschließend aus Methanol/Diethylether umkristallisiert.
Ausbeute: 1,73 g (=93 % d. Th.)
DC: Rf-Wert: 0,19 (Laufmittel analog Stufe II.1); Smp.: 224-225°C.
C₂₃H₂₄F₂NO₄xBr (496,35);

| | | | | |
|---|---|---|---|---|
| Elementaranalyse: | berechnet: | C (55,66) | H (4,87) | N (2,82) |
| | gefunden.: | C (55,20) | H (4,81) | N (2,82), |

### Beispiel 9: 2,2',4,4'-Tetrafluorbenzilsäuretropenolester-Methobromid (nicht gemäß der Erfindung) :

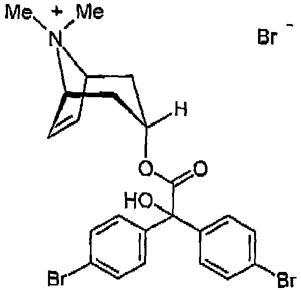

1,50 g (0,003 mol) der freien Base von **4.9** werden analog zu Beispiel 1 umgesetzt. Die entstandenen Kristalle werden aus Methanol umkristallisiert.
Ausbeute: 1,53 g (=86 % d. Th.)
DC: Rf-Wert: 0,14 (Laufmittel analog Stufe II.1); Smp.: 175-177°C.
C₂₃H₂₄Br₂NO₃xBr (602,16);

| | | | | |
|---|---|---|---|---|
| Elementaranalyse: | berechnet: | C (45,88) | H (4,02) | N (2,33) |
| | gefunden.: | C (45,46) | H (4,42) | N (2,18). |

### Beispiel 10: 4,4'-Dimethylbenzilsäuretropenolester-Methobromid

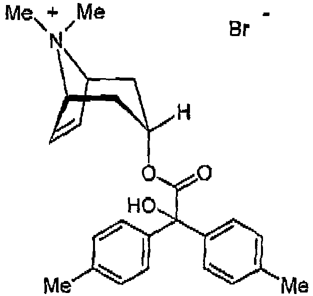

2,60 g (0,007 mol) der freien Base von **4.10** werden analog zu Beispiel 1 umgesetzt. Die entstandenen Kristalle werden aus Ethanol umkristallisiert.
Ausbeute: 3,16 g (=97 % d. Th.)
DC: Rf-Wert; 0,14 (Laufmittel analog Stufe II.1);
C₂₅H₃₀NO₃xBr (472,42);

| | | | | |
|---|---|---|---|---|
| Elementaranalyse: | berechnet: | C (63,56) | H (6,40) | N (2,96) |
| | gefunden.: | C (62,88) | H (6,87) | N (2,74). |

### Beispiel 11:4,4'-Dimethylbenzilsäurescopinester-Methobromid (nicht gemäß der Erfindung) :

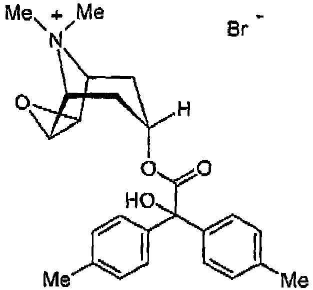

0,56 g (0,0014 mol) der freien Base von **4.11** werden analog zu Beispiel 1 umgesetzt. Die entstandenen Kristalle werden aus Methanol/Diethylether umkristallisiert.
Ausbeute: 0,55 g (=80 % d. Th.)
DC: Rf-Wert. 0,19 (Laufmittel analog Stufe II.1); Smp.: 221-222°C;
C₂₅H₃₀NO₄xBr (488,42);

| | | | | |
|---|---|---|---|---|
| Elementaranalyse: | berechnet: | C (61,48) | H (6,19) | N (2,87) |
| | gefunden.: | C (60,61) | H (6,31) | N (2,80). |

### Beispiel 13:3,3',4,4-Tetramethoxybenzilsäuretropenolester-Methobromid (nicht gemäß der Erfindung) :

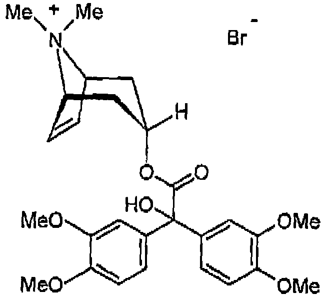

1,20 g (0,003 mol) 4.13 werden analog zu Beispiel 1 umgesetzt. Die entstandenen Kristalle werden aus Acetonitril/Diethylether umkristallisiert,
Ausbeute: 1,05 g (=73 % d. Th.)
DC: Rf-Wert: 0,10 (Laufmittel analog Stufe 11.1); Smp.: 212°C;
C₂₇H₃₄NO₇xBr (564,47);

| | | | | |
|---|---|---|---|---|
| Elementaranalyse: | berechnet: | C (57,45) | H (6,07) | N (2,48) |
| | gefunden.: | C (56,91) | H (6,05) | N (2,45). |

### Beispiel 14: 4,4'-Dimethoxybenzilsäuretropenolester-Methobromid (nicht gemäß der Erfindung) :

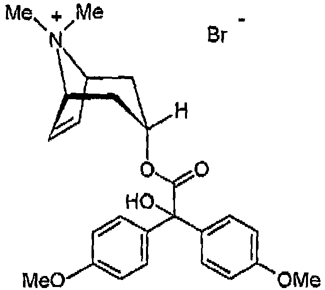

1,13 g (0,003 mol) 4.14 werden analog zu Beispiel 1 umgesetzt. Die entstandenen Kristalle werden aus Acetonitril/Diethylether umkristallisiert.
Ausbeute: 1,21 g (=87 % d. Th.)
C₂₅H₃₀NO₅xBr (504,42);
DC: Rf-Wert: 0,01 (Laufmittel analog Stufe II.1); Smp.: 180-181°C;

| | | | | |
|---|---|---|---|---|
| Elementaranalyse: | berechnet: | C (59,53) | H (5,99) | N (2,78) |
| | gefunden.: | C (59,29) | H (6,24) | N (2,84). |

### Beispiel 16: 3,3',4,4'-Tetramethoxybenzilsäurescopinester-Methobromid (nicht gemäß der Erfindung):

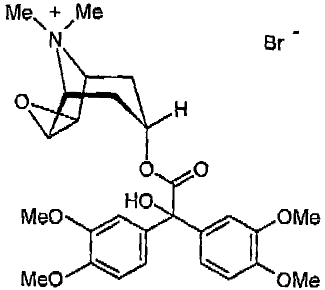

0,80 g (0,0017 mol) **4.16** werden analog zu Beispiel 1 umgesetzt. Die entstandenen Kristalle werden aus Aceton umkristallisiert.
Ausbeute: 0,35 g (=37 % d. Th.); Smp.: 211-212°C;
C₂₇H₃₄NO₈xBr (580,47);

| | | | | |
|---|---|---|---|---|
| Elementaranalyse: | berechnet: | C (55,87) | H (5,90) | N (2,41) |
| | gefunden.: | C (55,62) | H (6,09) | N (2,53). |

### Beispiel 17:3,3'-Dimethyl-4,4'-Dimethoxybenzilsäuretropenolester-Methobromid (nicht gemäß der Erfindung) :

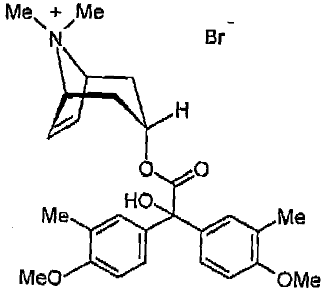

1,00 g (0,003 mol) 4.17 werden analog zu Beispiel 1 umgesetzt.
Ausbeute: 0,85 g (=70 % d. Th.); Smp.: 217°C;
C₂₇H₃₄NO₅xBr (532,47);

| | | | | |
|---|---|---|---|---|
| Elementaranalyse: | berechnet: | C (60,90) | H (6,51) | N (2,63) |
| | gefunden.: | C (59,83) | H (6,51) | N (2,93). |

### Beispiel 18: 3,3'-Dimethyl-4,4'-Dimethoxybenzilsäurescopinester (nicht gemäß der Erfindung) :

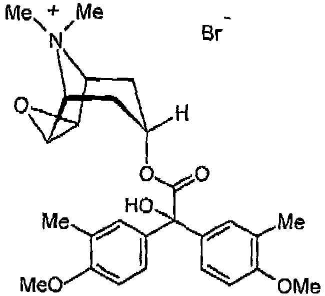

0,60 g (0,0013 mol) **4.18** werden analog zu Beispiel 1 umgesetzt. Die Reingigung erfolgt durch Kristallisation aus Aceton;
Ausbeute: 0,40 g (=56 % d. Th.);
C₂₇H₃₄NO₆xBr (548,47);

| | | | | |
|---|---|---|---|---|
| Elementaranalyse: | berechnet: | C (59,13) | H (6,25) | N (2,55) |
| | gefunden.: | C (58,69) | H (6,54) | N (2,61). |

### Beispiel 19: 3,3',4,4'-Tetrafluorbenzilsäuretropenolester-Ethylbromid :

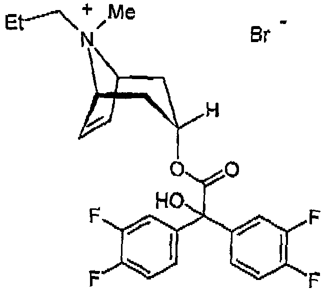

2,00 g (0,0048 mol) **4.1** wird in 20 ml Dichlormethan und 20 ml Acetonitril gelöst, mit 2,59 g (0,0238 mol) Bromethan versetzt und das Reaktionsgefäß verschlossen. Das Reaktionsgefäß wird unter Lichtausschluß bei etwa 20°C 3 Wochen stehen gelassen. Die Lösung wird zur Trockene eingedampft und der Rückstand aus Acetonitril umkristallisiert.
Ausbeute: 1,96 g 3 (= 78 % d. Th.)
DC: Rf-Wert: 0,11 (Laufmittel analog Stufe II.1); Smp.: 247°C.

| | | | | |
|---|---|---|---|---|
| Elementaranalyse: | berechnet: | C (54,35) | H (4,56) | N (2,64) |
| | gefunden.: | C (53,93) | H (4,59) | N (2,60). |

### Beispiel 20: 3,3',5,5'-Tetrafluorbenzilsäuretropenolester-Methobromid :

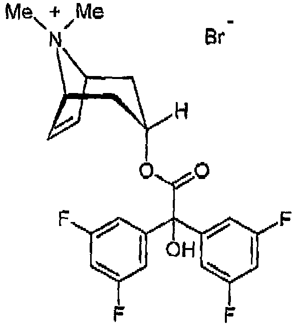

2,0 g (0,0047 mol) **4.19** werden analog zu Beispiel 1 umgesetzt.
Ausbeute: 2,22 g weiße Kristalle (=92 % d. Th.); Smp.: 262-264°C;
C₂₃H₂₂F₄NO₃xBr (516,33);

| | | | | |
|---|---|---|---|---|
| Elementaranalyse: | berechnet: | C (53,50) | H (4,29) | N (2,71) |
| | gefunden.: | C (53,48) | H (4,30) | N (2,65). |

### Beispiel 21: 2,2',4,4'-Tetrafluorbenzilsäurescopinester-Methobromid :

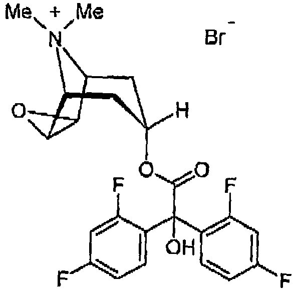

1,05 g (0,0024 mol) **4.20** werden analog zu Beispiel 1 umgesetzt.
Ausbeute: 0,54 g weiße Kristalle (=42 % d. Th.); Smp.: 208-209°C;
C₂₃H₂₂F₄NO₄xBr (532,33);

| | | | | |
|---|---|---|---|---|
| Elementaranalyse: | berechnet: | C (51,90) | H (4,17) | N (2,63) |
| | gefunden.: | C (50,84) | H (4,39) | N (2,50). |

### Beispiel 22: 3,3',5,5'-Tetrafluorbenzilsäurescopinester-Methobromid :

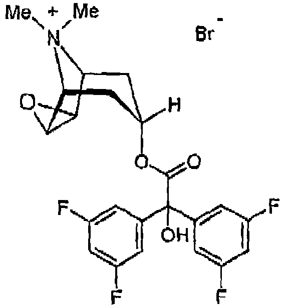

1,13 g (0,00258 mol) **4.21** werden analog zu Beispiel 1 umgesetzt.
Ausbeute: 1,07 g weiße Kristalle (=78 % d. Th.); Smp.: 238-239°C;
C₂₃H₂₂F₄NO₄xBr (532,33);

| | | | | |
|---|---|---|---|---|
| Elementaranalyse: | berechnet: | C (51,90) | H (4,17) | N (2,63) |
| | gefunden.: | C (51,85) | H (4,29) | N (2,70). |

### Beispiel 23: 3.3'-Dichlorbenzilsäuretropenolester-Methobromid (nicht gemäß der Erfindung) :

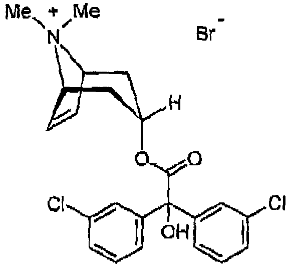

2,09 g (0,005 mol) **4.22** werden analog zu Beispiel 1 umgesetzt.
Ausbeute: 2,42 g weiße Kristalle (=94 % d. Th.); Smp.: 200-201°C;
C₂₃H₂₄Cl₂NO₃xBr (513,26);

| | | | | |
|---|---|---|---|---|
| Elementaranalyse: | berechnet: | C (53,82) | H (4,71) | N (2,73) |
| | gefunden.: | C (53,73) | H (4,74) | N (2,78). |

### Beispiel 24: 4,4'-Di(trifluormethybenzilsäuretropenylester-Methobromid (nicht gemäß der Erfindung) :

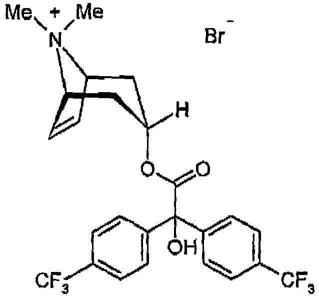

1,2 g (0,00247 mol) **4.23** werden analog zu Beispiel 1 umgesetzt.
Ausbeute: 1,05 g weiße Kristalle (=73 % d. Th.); Smp.: 140-141°C;
C₂₅H₂₄F₆NO₃xBr (580,36);

### Beispiel 25: 3,3'-Di(trifluormethyl)benzilsäuretropenolester-Methobromid (nicht gemäß der Erfindung) :

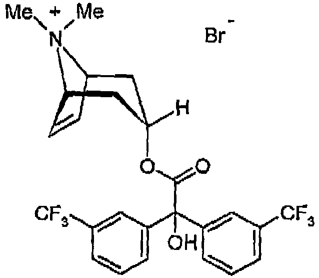

1,09 g (0,00225 mol) **4.24** werden analog zu Beispiel 1 umgesetzt.
Ausbeute: 0,84 g weiße Kristalle (=65 % d. Th.); Smp.: 228-229°C;
C₂₅H₂₄F₆NO₃xBr (580,36);

| | | | | |
|---|---|---|---|---|
| Elementaranalyse: | berechnet: | C (51,74) | H (4,17) | N(2,41) |
| | gefunden.: | C(51,40) | H (4,24) | N(2,42). |

### Beispiel 26: 4,4'-Di(trifluormethyl)benzilsäurescopinester-Methobromid (nicht gemäß der Erfindung) :

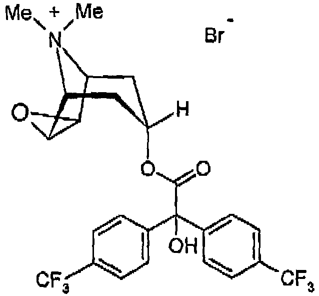

0,95 g (0,0019 mol) **4.25** werden analog zu Beispiel 1 umgesetzt. Das Produkt wird aus Diethylether umkristallisiert. Ausbeute: 0,98 g weiße Kristalle (=87 % d. Th.);
Smp._{:} 158-160°C; C₂₅H₂₄F₆NO₄xBr (596,36);

| | | | | |
|---|---|---|---|---|
| Elementaranalyse: | berechnet: | C (50,35) | H (4,06) | N (2,35) |
| | gefunden.: | C (50,34) | H (4,03) | N (2,36). |

### Beispiel 27: 3,3'-Di(trifluormethyl)benzilsäurescopinester-Methobromid (nicht gemäß der Erfindung) :

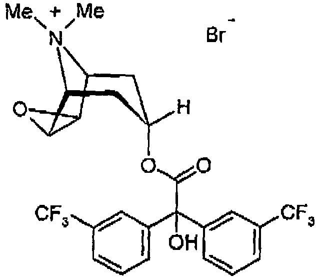

1,0 g (0,002 mol) **4.26** werden analog zu Beispiel 1 umgesetzt, Das Produkt wird aus Acetonitril umkristallisiert. Ausbeute: 0,7 g weiße Kristalle (=59 % d. Th.);
Smp.: 220-221°C; C₂₅H₂₄F₆NO₄xBr (596,36);

| | | | | |
|---|---|---|---|---|
| Elementaranalyse: | berechnet: | C (50,35) | H (4,06) | N (2,35) |
| | gefunden.: | C (50,24) | H (4,17) | N (2,40). |

### Beispiel 28: 4,4'-Difluorbenzilsäuretropenolester-Ethylbromid :

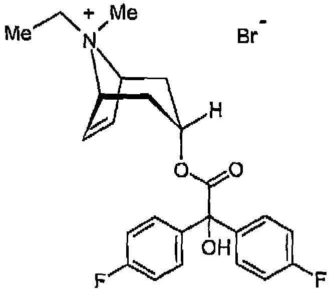

1,54 g (0,004 mol) **4.5** werden analog zu Beispiel 19 umgesetzt. Das Produkt wird aus Ethanol umkristallisiert. Ausbeute: 1,72 g weiße Kristalle (=87 % d. Th.);
Smp.: 228-229°C; C₂₄H₂₆F₂NO₃xBr (494,37);

| | | | | |
|---|---|---|---|---|
| Elementaranalyse: | berechnet: | C (58,31) | H (5,30) | N (2,83) |
| | gefunden.: | C (58,25) | H (5,29) | N (2,83). |

### Beispiel 29: 3,3'-Dichlor-4,4'-difluorbenzilsäuretropenolester-Methobromid (nicht gemäß der Erfindung) :

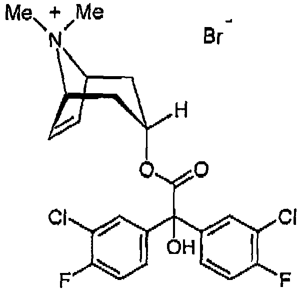

1,5 g (0,0033 mol) **4.27** werden analog zu Beispiel 1 umgesetzt. Das Produkt wird aus Acetonitril umkristallisiert. Ausbeute: 1,49 g weiße Kristalle (=82 % d. Th.);
Smp.: 245-246°C; C₂₃H₂₂Cl₂F₂NO₃xBr (549,24);

| | | | | |
|---|---|---|---|---|
| Elementaranalyse: | berechnet: | C (50,30) | H (4,04) | N (2,55) |
| | gefunden.: | C (50,44) | H (4,19) | N(2,51). |

### Beispiel 30: 3,3'-Dichlorbenzilsäurescopinester-Methobromid (nicht gemäß der Erfindung):

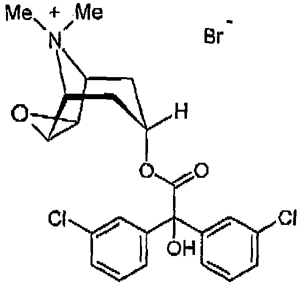

2,61 g (0,006 mol) **4.28** werden analog zu Beispiel 1 umgesetzt. Das Produkt wird aus Ethanol umkristallisiert. Ausbeute: 2,13 g weiße Kristalle (=67 % d, Th.);
Smp.: 221-222°C; C₂₃H₂₄Cl₂NO₄xBr (529,26);

| | | | | |
|---|---|---|---|---|
| Elementara,nalyse: | berechnet: | C (52,20) | H (4,57) | N (2,65) |
| | gefunden.: | C (52,25) | H (4,61) | N (2,70). |

### Beispiel 31: 2,2',5,5'-Tetrafluorbenzilsäuretropenolester-Methobromid :

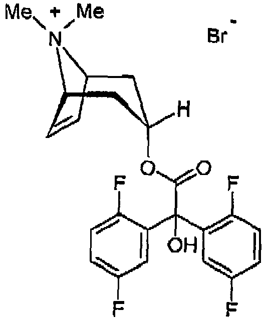

0,67 g (0,0016 mol) **4.29** werden analog zu Beispiel 1 umgesetzt. Das Produkt wird aus Methanol/Diethylether umkristallisiert,
Ausbeute: 0,70 g weiße Kristalle (=86 % d. Th.);
Smp.: 269-270°C; C₂₃H₂₂F₄NO₃xBr (516,33);

| | | | | |
|---|---|---|---|---|
| Elementaranalyse: | berechnet: | C (53,50) | H (4,29) | N (2,71) |
| | gefunden.: | C (53,30) | H (4,52) | N (2,76). |

### Beispiel 32: 2,2',3,3'-Tetrafluorbenzilsäuretropenolester-Methobromid:

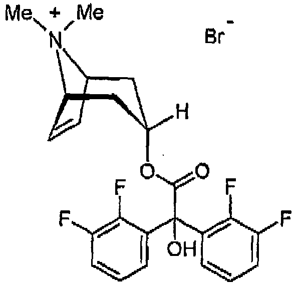

0,96 g (0,002 mol) **4.30** werden analog zu Beispiel 1 umgesetzt. Das Produkt wird aus Acetonitril umkristallisiert.
Ausbeute: 0,61 g weiße Kristalle (=59 % d. Th.);
Smp.: 268-269°C; C₂₃H₂₂F₄NO₃xBr (516,33);

| | | | | |
|---|---|---|---|---|
| Elementaranalyse: | berechnet: | C (53,50) | H (4,29) | N (2,71) |
| | gefunden.: | C (53,56) | H (4,38) | N (2,75). |

### Beispiel 33: 4,4'-Difluorbenzilsäurescopinester-Ethylbromid :

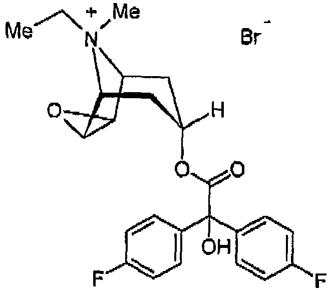

1,2 g (0,003 mol) **4.8** werden analog zu Beispiel 19 umgesetzt. Das Produkt wird aus Methanol umkristallisiert. Ausbeute: 0,93 g weiße Kristalle (=61 % d. Th.);
Smp.: 162-163°C; C₂₄H₂₆F₂NO₄xBr (510,38);

| | | | | |
|---|---|---|---|---|
| Elementaranalyse: | berechnet: | C (56,48) | H (5,13) | N (2,74) |
| | gefunden.: | C (55,96) | H (5,30) | N (2,75). |

### Beispiel 34: 3,3'-Difluorbenzilsäuretropenolester Methobromid :

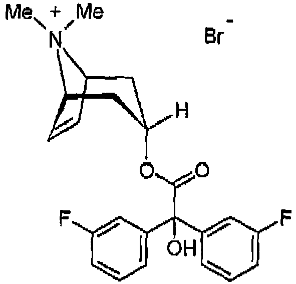

1,61 g (0,004 mol) **4.31** werden analog zu Beispiel 1 umgesetzt. Das Produkt wird aus Ethanol/Diethylether umkristallisiert.
Ausbeute: 1,93 g weiße Kristalle (=96 % d. Th.);
Smp.: 227-228°C; C₂₃H₂₄F₂NO₃xBr (480,35);

| | | | | |
|---|---|---|---|---|
| Elementaranalyse: | berechnet: | C (57,51) | H (5,04) | N (2,92) |
| | gefunden.: | C (57,38) | H (5,14) | N (2,95). |

### Beispiel 35: 3,3'-Difluorbenzilsäurescopinester-Methobromid :

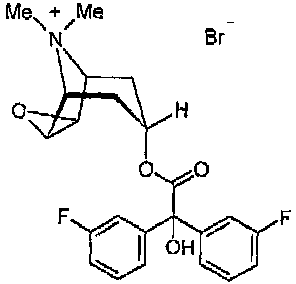

1,61 g (0,004 mol) **4.32** werden analog zu Beispiel 1 umgesetzt. Das Produkt wird aus Ethanol umkristallisiert.
Ausbeute: 1,83 g weiße Kristalle (=92 % d. Th.);
Smp.: 221-222°C; C₂₃H₂₄F₂NO₄xBr (496,35);

| | | | | |
|---|---|---|---|---|
| Elementaranalyse: | berechnet: | C (55,66) | H (4,87) | N (2,82) |
| | gefunden.: | C (55,49) | H (4,78) | N (2,73). |

### Beispiel 36: 3,3'-Dichlor-4,4'-difluorbenzilsäurescopinester-Methobromid (nicht gemäß der Erfindung) :

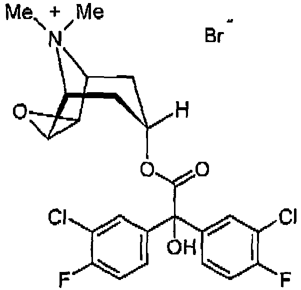

0,78 g (0,002 mol) **4.33** werden analog zu Beispiel 1 umgesetzt. Das Produkt wird aus Acetonitril umkristallisiert.
Ausbeute: 0,67 g weiße Kristalle (=59 % d. Th.);
C₂₃H₂₂Cl₂F₂NO₄xBr (565,24);

| | | | | |
|---|---|---|---|---|
| Elementaranalyse: | berechnet: | C (48,87) | H (3,92) | N (2,48) |
| | gefunden.: | C (48,87) | H (3,81) | N (2,46). |

### Beispiel 37: 4,4'-Dichlor-3,3-difluorbenzilsäurescopinester-Methobromid (nicht gemäß der Erfindung) :

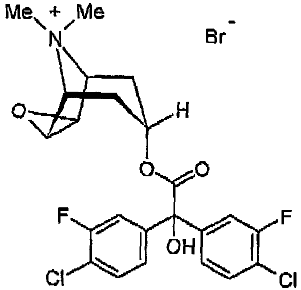

2,4 g (0,0051 mol) **4.35** werden analog zu Beispiel 1 umgesetzt. Das Produkt wird aus Acetonitril umkristallisiert. Ausbeute: 2,45 g weiße Kristalle (=85 % d. Th.); Smp.: 211-212°C; C₂₃H₂₂Cl₂F₂NO₄xBr (565,24).

### Beispiel 38: 3,3',4,4'-Tetrachlorbenzilsäuretropenolester-Methobromid (nicht gemäß der Erfindung) :

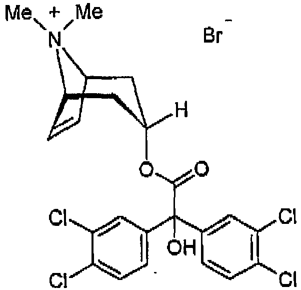

1,8 g (0,00369 mol) **4.36** werden analog zu Beispiel 1 umgesetzt. Das Produkt wird aus Methanol/Diethylether umkristallisiert.
Ausbeute: 2,01 g weiße Kristalle (=93 % d. Th.);
Smp.: 245-246°C; C₂₃H₂₂Cl₄NO₃xBr (582,15);

| | | | | |
|---|---|---|---|---|
| Elementaranalyse: | berechnet: | C (47,45) | H (3,81) | N (2,41) |
| | gefunden.: | C (47,27) | H (3,82) | N (2,36). |

### Beispiel 39: 4,4-Dichlor-3,3'-difluorbenzilsäuretropenolester-Methobromid (nicht gemäß der Erfindung) :

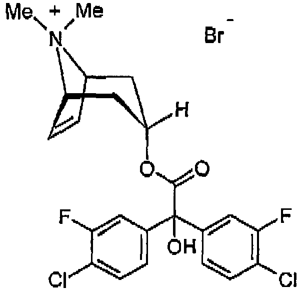

2,5 g (0,0055 mol) **4.34** werden analog zu Beispiel 1 umgesetzt. Das Produkt wird aus Acetonitril umkristallisiert. Ausbeute: 1,53 g weiße Kristalle (=51 % d. Th.); Smp.: 229-231°C; C₂₃H₂₂Cl₂F₂NO₃xBr (549,24).

### Beispiel 40: 3,3',4,4',5,5'-Tetrafluorbenzilsäuretropenolester-Methobromid :

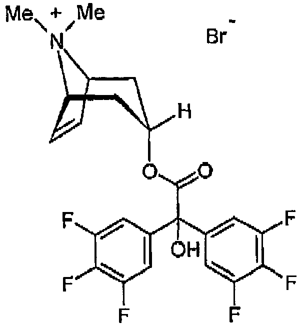

1,7 g (0,0037 mol) **4.37** werden analog zu Beispiel 1 umgesetzt. Das Produkt wird aus Aceton ausgerührt. Ausbeute: 1,9 g weiße Kristalle (=92 % d. Th.);
Smp.: 241-242°C; C₂₃H₂₀F₆NO₃xBr (552,32).

| | | | | |
|---|---|---|---|---|
| Elementaranalyse: | berechnet: | C (50,02) | H (3,65) | N (2,54) |
| | gefunden.: | C (50,09) | H (3,61) | N (2,49). |

Wie gefunden wurde, zeichnen sich die Verbindungen der allgemeinen Formel **1** durch vielfältige Anwendungsmöglichkeiten auf therapeutischem Gebiet aus. Hervorzuheben sind solche Anwendungsmöglichkeiten, für welche die erfindungsgemäßen Verbindungen der Formel **1** aufgrund ihrer pharmazeutischen Wirksamkeit als Anticholinergikum bevorzugt zur Anwendung gelangen können. Dies sind beispielsweise die Therapie von Asthma oder COPD (chronic obstructive pulmonary disease = chronisch obstruktive Lungenerkrankung). Die Verbindungen der allgemeinen Formel **1** können ferner zur Behandlung vagal bedingter Sinusbradykardien und zur Behandlung von Herz-Rhythmus-Störungen zum Einsatz gelangen. Generell lassen sich die erfindungsgemäßen Verbindungen ferner zur Behandlung von Spasmen beispielsweise im Gastrointestinaltrakt mit therapeutischem Nutzen einsetzen. Sie können ferner bei der Behandlung von Spasmen in harnableitenden Wegen sowie beispielsweise bei Menstruationsbeschwerden zum Einsatz gelangen.
Von den vorstehend beispielhaft aufgeführten Indiaktionsgebieten, kommt der Therapie von Asthma und COPD mittels der erfindungsgemäßen Verbindungen der Formel **1** ein besondere Bedeutung zu.

Die Verbindungen der allgemeinen Formel **1** können allein oder in Kombination mit anderen erfindungsgemäßen Wirkstoffen der Formel **1** zur Anwendung gelangen. Gegebenenfalls können die Verbindungen der allgemeinen Formel **1** auch in Kombination mit weiteren pharmakologisch aktiven Wirkstoffen eingesetzt werden. Es handelt sich hierbei insbesondere um Betamimetica, Antiallergika, PAF-Antagonisten, Leukotrien-Antagonisten und Corticosteroiden, sowie Wirkstoffkombinationen davon.

Als Beispiel für Betamimetika, die erfindungsgemäß mit den Verbindungen der Formel **1** als Kombination zum Einsatz kommen können, seien genannt Verbindungen, die ausgewählt sind aus der Gruppe bestehend aus Bambuterol, Bitolterol, Carbuterol, Clenbuterol, Fenoterol, Formoterol, Hexoprenalin, Ibuterol, Pirbuterol, Procaterol, Reproterol, Salmeterol, Sulfonterol, Terbutalin, Tolubuterol, 4-Hydroxy-7-[2-{[2-{[3-(2-phenylethoxy)propyl]sulfonyl}ethyl]-amino}ethyl]-2(3H)-benzothiazolon, 1-(2-Fluoro-4-hydroxyphenyl)-2-[4-(1-benzimidazolyl)-2-methyl-2-butylamino]ethanol, 1-[3-(4-Methoxybenzyl-amino)-4-hydroxyphenyl]-2-[4-(1-benzimidazolyl)-2-methyl-2-butylamino]ethanol, 1-[2H-5-Hydroxy-3-oxo-4H-1,4-benzoxazin-8-yl]-2-[3-(4-N,N-dimethylaminophenyl)-2-methyl-2-propylamino]ethanol, 1-[2H-5-Hydroxy-3-oxo-4H-1,4-benzoxazin-8-yl]-2-[3-(4-methoxyphenyl)-2-methyl-2-propylamino]ethanol, 1-[2H-5-Hydroxy-3-oxo-4H-1,4-benzoxazin-8-yl]-2-[3-(4-n-butyloxyphenyl)-2-methyl-2-propylamino]ethanol, 1-[2H-5-Hydroxy-3-oxo-4H-1,4-benzoxazin-8-yl]-2-{4-[3-(4-methoxyphenyl)-1,2,4-triazol-3-yl]-2-methyl-2-butylamino}ethanol, 5-Hydroxy-8-(1-hydroxy-2-isopropylaminobutyl)-2H-1,4-benzoxazin-3-(4H)-on, 1-(4-Amino-3-chloro-5-trifluormethylphenyl)-2-*tert*.-butylamino)ethanol und 1-(4-Ethoxycarbonylamino-3-cyano-5-fluorophenyl)-2-(*tert.-*butylamino)ethanol, gegebenenfalls in Form ihrer Racemate, ihrer Enantiomere, ihrer Diastereomere, sowie gegebenenfalls ihrer pharmakologisch unbedenklichen Säureadditionssalze und Hydrate. Besonders bevorzugt gelangen als Betamimetika solche Wirkstoffe in Kombination mit den erfindungsgemäßen Verbindungen der Formel **1** zur Anwendung, die ausgewählt sind aus der Gruppe bestehend aus Fenoterol, Formoterol, Salmeterol, 1-[3-(4-Methoxybenzyl-amino)-4-hydroxyphenyl]-2-[4-(1-benzimidazolyl)-2-methyl-2-butylamino]ethanol, 1-[2H-5-Hydroxy-3-oxo-4H-1,4-benzoxazin-8-yl]-2-[3-(4-N,N-dimethylaminophenyl)-2-methyl-2-propylamino]ethanol, 1-[2H-5-Hydroxy-3-oxo-4H-1,4-benzoxazin-8-yl]-2-[3-(4-methoxyphenyl)-2-methyl-2-propylamino]ethanol, 1-[2H-5-Hydroxy-3-oxo-4H-1,4-benzoxazin-8-yl]-2-[3-(4-n-butyloxyphenyl)-2-methyl-2-propylamino]ethanol, 1-[2H-5-Hydroxy-3-oxo-4H-1,4-benzoxazin-8-yl]-2-{4-(3-(4-methoxyphenyl)-1,2,4-triazol-3-yl]-2-methyl-2-butylamino}ethanol, gegebenenfalls in Form ihrer Racemate, ihrer Enantiomere, ihrer Diastereomere, sowie gegebenenfalls ihrer pharmakologisch unbedenklichen Säureadditionssalze und Hydrate, Von den vorstehend genannten Betamimetika kommt hierbei den Verbindungen Formoterol und Salmeterol gegebenenfalls in Form ihrer Racemate, ihrer Enantiomere, ihrer Diastereomere, sowie gegebenenfalls ihrer pharmakologisch unbedenklichen Säureadditionssalze und Hydrate besondere Bedeutung zu.
Erfindungsgemäß bevorzugt sind die Säureadditionssalze der Betamimetika ausgewählt aus der Gruppe bestehend aus Hydrochlorid, Hydrobromid, Sulfat, Phosphat, Fumarat Methansulfonat und Xinafoat. Besonders bevorzugt sind die Salze im Falle des Salmeterols ausgewählt aus Hydrochlorid, Sulfat und Xinafoat, von denen die Sulfate und Xinafoate besonders bevorzugt sind. Erfingdungsgemäß von herausragender Bedeutung sind Salmeterol x ½ H₂SO₄ und Salmeterolxinafoat. Besonders bevorzugt sind die Salze im Falle des Formoterols ausgewählt aus Hydrochlorid, Sulfat und Fumarat, von denen das Hydrochlorid und Fumarat besonders bevorzugt sind. Erfindungsgemäß von herausragender Bedeutung ist Formoterolfumarat.

Im Rahmen der vorliegenden Erfindung werden unter Corticosteroiden, die gegebenenfalls in Kombination mit den Verbindungen der Formel **1** zum Einsatz gelangen können, Verbindungen verstanden, die ausgewählt sind aus der Gruppe bestehend aus Flunisolide, Beclomethasone, Triamcinolone, Budesonid, Fluticasone, Mometasone, Ciclesonide, Rofleponide, GW 215864, KSR 592, ST-126 und Dexametasone. Bevorzugt sind im Rahmen der vorliegenden Erfindung die Corticosteroide ausgewählt aus der Gruppe bestehend aus Flunisolide, Beclomethasone, Triamcinolone, Budesonid, Fluticasone, Mometasone, Ciclesonide und Dexametasone, wobei hier dem Budesonid, Flutleasone, Mometasone und Ciciesonide, insbesondere dem Budesonid und dem Fluticason eine besondere Bedeutung zukommt. Gegebenfalls wird im Rahmen der vorliegenden Patentanmeldung statt der Bezeichnung Corticosteroide auch nur die Bezeichnung Steroide verwendet. Eine Bezugnahme auf Steroide schließt im Rahmen der vorliegenden Erfindung eine Bezugnahme auf Salze oder Derivate, die von den Steroiden gebildet werden können, mit ein. Als mögliche Salze oder Derivate werden beispielsweise genannt: Natriumsalze, Sulfobenzoate, Phosphate, Isonicotinate, Acetate, Propionate, Dihydrogenphosphate, Palmitate, Pivalate oder Furoate. Gegebenenfalls können die Corticosteroide auch in Form ihrer Hydrate vorliegen.

Im Rahmen der vorliegenden Erfindung werden unter Dopamin-Agonisten, die gegebenenfalls in Kombination mit den Verbindungen der Formel **1** zum Einsatz gelangen können, Verbindungen verstanden, die ausgewählt sind aus der Gruppe bestehend aus Bromocriptin, Cabergolin, Alpha-Dihydroergocryptin, Lisurid, Pergolid, Pramipexol, Roxindol, Ropinirol, Talipexol, Tergurid und Viozan. Bevorzugt werden im Rahmen der vorliegenden Erfindung Dopamin-Agonisten als Kombinationspartner mit den Verbindungen der Formel **1** eingesetzt, die ausgewählt sind aus der Gruppe bestehend aus Pramipexol, Talipexol und Viozan, wobei Pramipexol eine besondere Bedeutung zukommt. Eine Bezugnahme auf die vorstehend genannten Dopamin-Agonisten schließt im Rahmen der vorliegenden Erfindung eine Bezugnahme auf deren gegebenenfalls existierende pharmakologisch verträgliche Säureadditionssalze und gegebenenfalls deren Hydrate ein. Unter den physiologisch verträglichen Säureadditionssalzen, die von den vorstehend genannten Dopaminagonisten gebildet werden können, werden beispielsweise pharmazeutisch verträgliche Salze verstanden, die ausgewählt aus den Salzen der Salzsäure, Bromwasserstoffsäure, Schwefelsäure, Phosphorsäure, Methansulfonsäure, Essigsäure, Fumarsäure, Bernsteinsäure, Milchsäure, Zitronensäure, Weinsäure und Maleinsäure sind.

Als Beispiel für Antiallergika, die erfindungsgemäß mit den Verbindungen der Formel **1** als Kombination zum Einsatz kommen können, seien genannt Epinastin, Cetirizin, Azelastin, Fexofenadin, Levocabastin, Loratadin, Mizolastin, Ketotifen, Emedastin, Dimetinden, Clemastin, Bamipin, Cexchlorpheniramin, Pheniramin, Doxylamin, Chlorphenoxamin, Dimenhydrinat, Diphenhydramin, Promethazin, Ebastin, Desloratidin und Meclozin. Bevorzugte Antiallergika, die im Rahmen der vorliegenden Erfindung in Kombination mit den erfindungsgemäßen Verbindungen der Formel **1** zum Einsatz gelangen können, sind ausgewählt aus der Gruppe bestehend aus Epinastin, Cetirizin, Azelastin, Fexofenadin, Levocabastin, Loratadin, Ebastin, Desloratidin und Mizolastin wobei Epinastin und Deslorstidin besonders bevorzugt sind. Eine Bezugnahme auf die vorstehend genannten Antiallergika schließt im Rahmen der vorliegenden Erfindung eine Bezugnahme auf deren gegebenenfalls existierende pharmakologisch verträgliche Säureadditionssalze ein.

Als Beispiel für PAF-Antagonisten, die erfindungsgemäß mit den Verbindungen der Formel **1** als Kombination zum Einsatz kommen können seien genannt 4-(2-Chlorphenyl)-9-methyl-2-[3(4-morpholinyl)-3-propanon-1-yl]-6H-thieno-[3,2-f] [1,2,4]triazolo[4,3-a][1,4]diazepin,
6-(2-Chlorphenyl)-8,9-dihydro-1-methyl-8-[(4-morpholinyl)carbonyl]-4H,7H-cyclo-penta-[4,5]thieno-[3,2-f][1,2,4]triazolo[4,3-a][1,4]diazepin.

Geeignete Anwendungsformen zur Applikation der Verbindungen der Formel **1** sind beispielsweise Tabletten, Kapseln, Zäpfchen, Lösungen etc.
Erfindungsgemäß von besonderer Bedeutung ist (insbesondere bei der Behandlung von Asthma oder COPD) die inhalative Applikation der erfindungsgemäßen Verbindungen. Der Anteil der pharmazeutisch wirksamen Verbindung(en) sollte jeweils im Bereich von 0,05 bis 90 Gew.-%, bevorzugt 0,1 bis 50 Gew.-% der Gesamtzusammensetzung liegen. Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen, wobei die oben bei den Tabletten erwähnten Hilfsstoffe verwendet werden können.
Säfte der erfindungsgemäßen Wirkstoffe beziehungsweise Wirkstoffkombinationen können zusätzlich noch ein Süßungsmittel, wie Saccharin, Cyclamat, Glycerin oder Zucker sowie ein geschmacksverbesserndes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Schutzstoffe, wie p-Hydroxybenzoate, enthalten.

Lösungen werden in üblicher Weise, z.B. unter Zusatz von Isotonantien, Konservierungsmitteln, wie p-Hydroxybenzoaten, oder Stabilisatoren, wie Alkalisalzen der Ethylendiamintetraessigsäure, gegebenenfalls unter Verwendung von Emulgiermitteln und /oder Dispergiermitteln, wobei beispielsweise bei der Verwendung von Wasser als Verdünnungsmittel gegebenenfalls organische Lösemittel als Lösevermittler bzw. Hilflösemittel eingesetzt werden können, hergestellt und in Injektionsflaschen oder Ampullen oder Infusionsflaschen abgefüllt.

Die eine oder mehrere Wirkstoffe beziehungsweise Wirkstoffkombinationen enthaltenden Kapseln können beispielsweise hergestellt werden, indem man die Wirkstoffe mit inerten Trägem, wie Milchzucker oder Sorbit, mischt und in Gelatinekapseln einkapselt.

Geeignete Zäpfchen lassen sich beispielsweise durch Vermischen mit dafür vorgesehenen Trägermitteln, wie Neutralfetten oder Polyäthylenglykol beziehungsweise dessen Derivaten, herstellen.
Als Hilfsstoffe seien beispielsweise Wasser, pharmazeutisch unbedenkliche organische Lösemittel, wie Paraffine (z.B. Erdölfraktionen), Öle pflanzlichen Ursprungs (z.B. Erdnuß- oder Sesamöl), mono- oder polyfunktionelle Alkohole (z.B. Ethanol oder Glycerin), Trägerstoffe wie z.B. natürliche Gesteinsmehle (z.B. Kaoline, Tonerden, Talkum, Kreide) synthetische Gesteinsmehle (z.B. hochdisperse Kieselsäure und Silikate), Zucker (z.B. Rohr-, Milch- und Traubenzucker) Emulgiermittel (z.B. Lignin, Sufitablaugen, Methylcellulose, Stärke und Polyvinylpyrrolidon) und Gleitmittel (z.B. Magnesiumstearat, Talkum, Stearinsäure und Natriumlaurylsulfat) erwähnt.

Die Applikation erfolgt in üblicher Weise, bei der Therapie von Asthma oder COPD vorzugsweise inhalativ.
Im Falle der oralen Anwendung können die Tabletten selbstverständlich außer den genannten Trägerstoffen auch Zusätze, wie z.B. Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen können die Wirkstoffe außer den obengenannten Hilfsstoffen mit verschiedenen Geschmacksaufbesserem oder Farbstoffen versetzt werden.

Die Dosierung der erfindungsgemäßen Verbindungen ist naturgemäß stark von der Applikationsart und der zu therapierenden Erkrankung abhängig. Bei inhalativer Applikation zeichnen sich die Verbindungen der Formel **1** bereits bei Dosen im µg-Bereich durch eine hohe Wirksamkeit aus. Auch oberhalb des µg-Bereichs, lassen sich die Verbindungen der Formel **1** sinnvoll einsetzen. Die Dosierung kann dann beispielsweise auch im Grammbereich liegen. Insbesondere bei nicht inhalativer Applikation können die erfindungsgemäßen Verbindungen mit höherer Dosierung appliziert werden (beispielsweise, aber nicht limitierend im Bereich von 1 bis 1000mg).

Die nachfolgenden Formulierungsbeipiele illustrieren die vorliegende Erfindung ohne sie jedoch in ihrem Umfang zu beschränken:

### Pharmazeutische Formulierungsbeispiele

| A) | Tabletten | pro Tablette |
|---|---|---|
| | Wirkstoff | 100 mg |
| | Milchzucker | 140 mg |
| | Maisstärke | 240 mg |
| | Polyvinylpyrrolidon | 15 mg |
| | Magnesiumstearat | 5 mg |
| | | 500 mg |

Der feingemahlene Wirkstoff, Milchzucker und ein Teil der Maisstärke werden miteinander vermischt. Die Mischung wird gesiebt, worauf man sie mit einer Lösung von Polyvinylpyrrolidon in Wasser befeuchtet, knetet, feuchtgranuliert und trocknet. Das Granulat, der Rest der Maisstärke und das Magnesiumstearat werden gesiebt und miteinander vermischt. Das Gemisch wird zu Tabletten geeigneter Form und Größe verpreßt.

| B) | Tabletten | pro Tablette |
|---|---|---|
| | Wirkstoff | 80 mg |
| | Milchzucker | 55 mg |
| | Maisstärke | 190 mg |
| | Mikrokristalline Cellulose | 35 mg |
| | Polyvinylpyrrolidon | 15mg |
| | Natrium-carboxymethylstärke | 23 mg |
| | Magnesiumstearat | 2 mg |
| | | 400 mg |

Der feingemahlene Wirkstoff, ein Teil der Maisstärke, Milchzucker, mikrokristalline Cellulose und Polyvinylpyrrolidon werden miteinander vermischt, die Mischung gesiebt und mit dem Rest der Maisstärke und Wasser zu einem Granulat verarbeitet, welches getrocknet und gesiebt wird. Dazu gibt man die Natriumcarboxymethylstärke und das Magnesiumstearat, vermischt und verpreßt das Gemisch zu Tabletten geeigneter Größe.

| C) | Ampullenlösung | |
|---|---|---|
| | Wirkstoff | 50 mg |
| | Natriumchlorid | 50 mg |
| | Aqua pro inj. | 5 ml |

Der Wirkstoff wird bei Eigen-pH oder gegebenenfalls bei pH 5,5 bis 6,5 in Wasser gelöst und mit Natriumchlorid als Isotonans versetzt. Die erhaltene Lösung wird pyrogenfrei filtriert und das Filtrat unter aseptischen Bedingungen in Ampullen abgefüllt, die anschließend sterilisiert und zugeschmolzen werden. Die Ampullen enthalten 5 mg, 25 mg und 50 mg Wirkstoff.

| D) | Dosieraerosol | |
|---|---|---|
| | Wirkstoff | 0,005 |
| | Sorbitantrioleat | 0,1 |
| | Monofluortrichlormethan und | |
| | Difluordichlormethan 2 : 3 | ad 100 |

Die Suspension wird in einen üblichen Aerosolbehälter mit Dosierventil gefüllt. Pro Betätigung werden vorzugsweise 50 µl Suspension abgegeben. Der Wirkstoff kann gewünschtenfalls auch höher dosiert werden (z.B. 0.02 Gew.-%).

| E) | Lösungen (in mg/100ml) | |
|---|---|---|
| | Wirkstoff | 333.3 mg |
| | Formoterolfumarat | 333.3 mg |
| | Benzalkoniumchlorid | 10.0 mg |
| | EDTA | 50,0 mg |
| | HCl (1n) | ad pH 3.4 |

Diese Lösung kann in üblicher Art und Weise hergestellt werden.

| F) | Inhalationpulver | |
|---|---|---|
| | Wirkstoff | 6 µg |
| | Formoterolfumarat | 6 µg |
| | Lactose Monohydrat | ad 25 mg |

Die Herstellung des Inhaltionspulvers erfolgt in üblicher Art und Weise durch Mischen der einzelnen Bestandteile.

| G) | Inhalationpulver | |
|---|---|---|
| | Wirkstoff | 10 µg |
| | Lactose Monohydrat | ad 5 mg |

Die Herstellung des Inhaltionspulvers erfolgt in üblicher Art und Weise durch Mischen der einzelnen Bestandteile.

## Patentansprüche

1. Verbindungen der allgemeinen Formel **1** 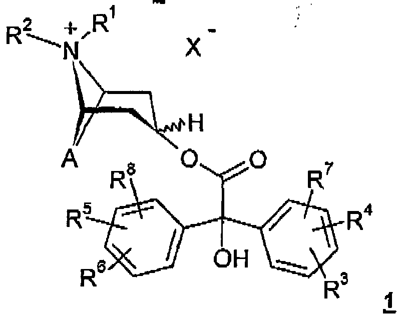 worin
A ein zweibindiger Rest ausgewählt aus der Gruppe bestehend aus 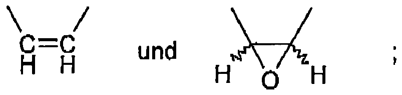
X⁻ ein einfach negativ geladenes Anion,
R¹ und R² C₁-C₄-Alkyl, welches gegebenenfalls durch Hydroxy oder Halogen substituiert sein kann;
R³, R⁴, R⁵, R⁶, R⁷ und R⁸, gleich oder verschieden, Wasserstoff oderFluor, mit der Maßgabe, daß wenigstens einer der Reste R³, R⁴, R⁵, R⁶, R⁷ und R⁸ nicht Wasserstoff ist,
bedeuten, gegebenenfalls in Form der einzeinen optischen isomeren, Mischungen der einzelnen Enantiomeren oder Racemate.

2. Verbindungen der allgemeinen Formel **1** nach Anspruch 1,
worin
A ein zweibindiger Rest ausgewählt aus der Gruppe bestehend aus 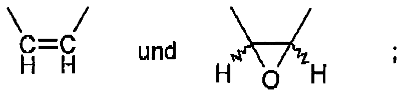
X⁻ ein einfach negativ geladenes Anion ausgewählt aus der Gruppe Chlorid, Bromid, Methylsulfat, 4-Toluolsulfonat und Methansulfonat;
R¹ und R² gleich oder verschieden ein Rest ausgewählt aus der Gruppe bestehend aus Methyl, Ethyl, n-Propyl und iso-Propyl, der gegebenenfalls durch Hydroxy oder Fluor substituiert sein kann;
R³, R⁴, R⁵, R⁶, R⁷ und R⁸, gleich oder verschieden, Wasserstoffoder Fluor, mit der Maßgabe, daß wenigstens einer der Reste R³, R⁴, R⁵, R⁶, R⁷ und R⁸ nicht Wasserstoff ist,
bedeuten, gegebenenfalls in Form der einzelnen optischen isomeren, Mischungen der einzelnen Enantiomeren oder Racemate.

3. Verbindungen der allgemeinen Formel **1** nach Anspruch 1 oder 2,
worin
A ein zweibindiger Rest ausgewählt aus der Gruppe bestehend aus 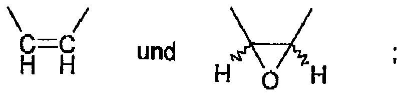
X⁻ Bromid;
R¹ und R² gleich oder verschieden Methyl oder Ethyl;
R³, R⁴, R⁵, R⁶, R⁷ und R⁸, gleich oder verschieden, Wasserstoff oder Fluor, mit der Maßgabe, daß wenigstens einer der Reste R³, R⁴, R⁵, R⁶, R⁷ und R⁸ nicht Wasserstoff ist, bedeuten, gegebenenfalls in Form der einzelnen optischen Isomeren, Mischungen der einzeinen Enantiomeren oder Racemate.

4. Verbindungen der allgemeinen Formel **1** nach Anspruch 1, 2 oder 3, worin
A ein zweibindiger Rest ausgewählt aus der Gruppe bestehend aus 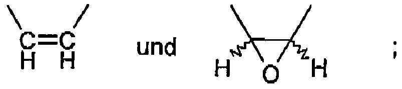
X⁻ Bromid;
R¹ und R² gleich oder verschieden Methyl oder Ethyl;
R³, R⁴, R⁵, R⁶, R⁷ und R⁸, gleich oder verschieden, Wasserstoff oder Fluor, mit der Maßgabe, daß wenigstens einer der Reste R³, R⁴, R⁵, R⁶, R⁷ und R⁸ nicht Wasserstoff ist,
bedeuten, gegebenenfalls in Form der einzelnen optischen Isomeren, Mischungen der einzelnen Enantiomeren oder Racemate.

5. Verbindungen der allgemeinen Formel **1** nach Anspruch 1, 2, 3 oder 4, worin
A ein zweibindiger Rest ausgewählt aus der Gruppe bestehend aus 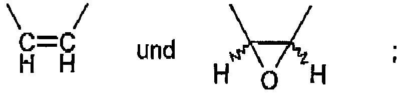
X⁻ Bromid;
R¹ und R² gleich oder verschieden Methyl oder Ethyl;
R³, R⁴, R⁵, R⁶, R⁷ und R⁸, gleich oder verschieden, Wasserstoff oder Fluor, mit der Maßgabe, daß wenigstens einer der Reste R³, R⁴, R⁵, R⁶, R⁷ und R⁸ nicht Wasserstoff ist,
bedeuten, gegebenenfalls in Form der einzelnen optischen Isomeren, Mischungen der einzelnen Enantiomeren oder Racemate.

6. Verbindungen der allgemeinen Formel **1** nach einem der Ansprüche 1 bis 5,
worin
A ein zweibindiger Rest ausgewählt aus der Gruppe bestehend aus 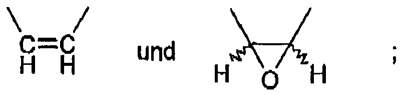
X⁻ Bromid;
R¹ und R² Methyl;
R³, R⁴, R⁵, R⁶, R⁷ und R⁸, gleich oder verschieden, Wasserstoff oder Fluor mit der Maßgabe, daß wenigstens einer der Reste R³, R⁴, R⁵, R⁶, R⁷ und R⁸ nicht Wasserstoff ist,
bedeuten, gegebenenfalls in Form der einzelnen optischen Isomeren, Mischungen der einzelnen Enantiomeren oder Racemate.

7. Verwendung einer Verbindung der allgemeinen Formel **1** gemäß einem der Ansprüche 1 bis 6 zur Herstellung eines Arzneimittels zur Behandlung von Erkrankungen, in denen Anticholinergika einen therapeutischen Nutzen entfalten können.

8. Verwendung einer Verbindung der allgemeinen Formel **1** gemäß einem der Ansprüchen 1 bis 6 zur Herstellung eines Arzneimittels zur Behandlung von Asthma, COPD, vagal bedingter Sinusbradykardien, Herz-Rhythmus-Störungen, Spasmen im Gastrointestinaltrakt, Spasmen in harnableitenden Wegen und Menstruationsbeschwerden.

9. Pharmazeutische Zubereitungen, enthaltend als Wirkstoff eine oder mehrere Verbindungen der allgemeinen Formel **1** gemäß einem der Ansprüche 1 bis 6 oder deren physiologisch verträgliche Salze gegebenenfalls in Kombination mit üblichen Hilfs- und/oder Trägerstoffen.

10. Pharmazeutische Zubereitungen nach Anspruch 9, **dadurch gekennzeichnet daß** diese neben einer oder mehrerer der Verbindungen der Formel **1** ferner wenigstens einen weiteren Wirkstoff enthalten, der ausgewählt ist aus der Gruppe der Betamimetica, Antiallergika, PAF-Antagonisten, Leukotrien-Antagonisten und Steroide.

11. Verfahren zur Herstellung einer Verbindung der allgemeinen Formel **1** 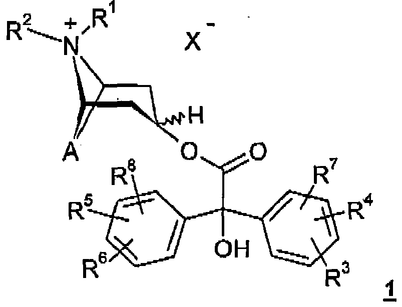 worin A, X⁻ und die Reste R¹, R², R³, R⁴, R⁵, R⁶, R⁷ und R⁸ die in den Ansprüchen 1 bis 6 genannten Bedeutungen haben können, **dadurch gekennzeichnet, daß** man in einem ersten Schritt eine Verbindung der allgemeinen Formel **3** 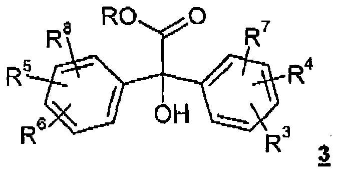 worin die Reste R³, R⁴, R⁵, R⁶, R⁷ und R⁸ die in den Ansprüchen 1 bis 6 genannten Bedeutungen haben können und R C₁-C₄-Alkyl bedeutet mit einer Verbindung der Formel **2** 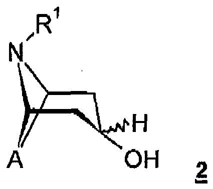 worin A und R¹ die in den Ansprüchen 1 bis 6 genannten Bedeutungen haben können zu einer Verbindung der Formel **4** 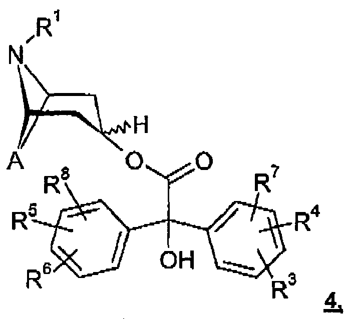 worin A und die Reste R¹, R³, R⁴, R⁵, R⁶, R⁷ und R⁸ die in den Ansprüchen 1 bis 6 genannten Bedeutungen haben können, umsetzt und diese anschließende durch Umsetzung mit einer Verbindung R²-X, worin R² und X die in den Ansprüchen 1 bis 6 genannten Bedeutungen haben können zu einer Verbindung der Formel **1** quarternisiert.

12. Zwischenprodukte der allgemeinen Formel **4** 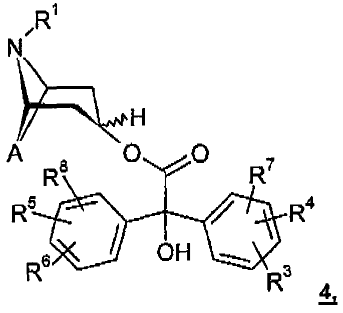 worin A und die Reste R¹, R³, R⁴, R⁵, R⁶, R⁷ und R⁸ die in den Ansprüchen 1 bis 6 genannten Bedeutungen haben können.

## Claims

1. Compounds of general formula **1** 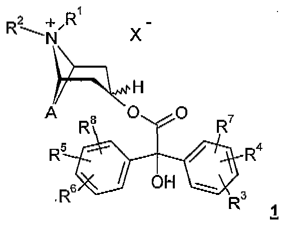 wherein
A denotes a double-bonded group selected from among 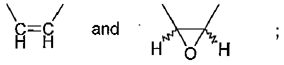
X⁻ denotes an anion with a single negative charge,
R¹ and R² denote C₁-C₄-alkyl which may optionally be substituted by hydroxy or halogen;
R³, R⁴, R⁵, R⁶, R⁷ and R⁸, which may be identical or different, denote hydrogen or fluorine, with the proviso that at least one of the groups R³, R⁴, R⁵, R⁶, R⁷ and R⁸ is not hydrogen,
optionally in the form of the individual optical isomers, mixtures of the individual enantiomers or racemates.

2. Compounds of general formula **1** according to claim 1, wherein
A denotes a double bonded group selected from among 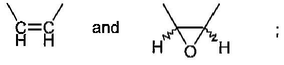
X⁻ denotes an anion with a single negative charge selected from among chloride, bromide, methylsulphate, 4-toluenesulphonate and methanesulphonate,
R¹ and R², which may be identical or different, denote a group selected from among methyl, ethyl, n-propyl and iso-propyl, which may optionally be substituted by hydroxy or fluorine;
R³, R⁴, R⁵, R⁶, R⁷ and R⁸, which may be identical or different, denote hydrogen or fluorine, with the proviso that at least one of the groups R³, R⁴, R⁵, R⁶, R⁷ and R⁸ is not hydrogen,
optionally in the form of the individual optical isomers, mixtures of the individual enantiomers or racemates.

3. Compounds of general formula **1** according to claim 1 or 2,
wherein
A denotes a double-bonded group selected from among 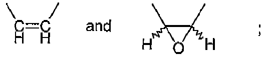
X⁻ denotes bromide;
R¹ and R², which may be identical or different, denote methyl or ethyl;
R³, R⁴, R⁵, R⁶, R⁷ and R⁸, which may be identical or different, denote hydrogen or fluorine, with the proviso that at least one of the groups R³, R⁴, R⁵, R⁶, R⁷ and R⁸ is not hydrogen,
optionally in the form of the individual optical isomers, mixtures of the individual enantiomers or racemates.

4. Compounds of general formula **1** according to claim 1, 2 or 3,
wherein
A denotes a double-bonded group selected from among 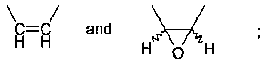
X⁻ denotes bromide;
R¹ and R², which may be identical or different, denote methyl or ethyl;
R³, R⁴, R⁵, R⁶, R⁷ and R⁸, which may be identical or different, denote hydrogen or fluorine, with the proviso that at least one of the groups R³, R⁴, R³, R⁶, R⁷ and R⁸ is not hydrogen,
optionally in the form of the individual optical isomers, mixtures of the individual enantiomers or racemates.

5. Compounds of general formula 1 according to claim 1, 2, 3 or 4, wherein
A denotes a double-bonded group selected from among 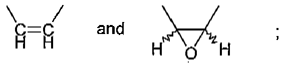
X⁻ denotes bromide;
R¹ and R², which may be identical or different, denote methyl or ethyl;
R³, R⁴, R⁵, R⁶, R⁷ and R⁸, which may be identical or different, denote hydrogen or fluorine, with the proviso that at least one of the groups R³, R⁴, R⁵, R⁶, R⁷ and R⁸ is not hydrogen,
optionally in the form of the individual optical isomers, mixtures of the individual enantiomers or racemates.

6. Compounds of general formula 1 according to one of claims 1 to 5, wherein
A denotes a double-bonded group selected from among 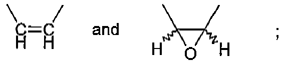
X⁻ denotes bromide;
R¹ and R² denote methyl;
R³, R⁴, R⁵, R⁶. R⁷ and R⁶, which may be identical or different, denote hydrogen or fluorine, with the proviso that at least one of the groups R³, R⁴, R⁵, R⁶, R⁷ and R⁸ is not hydrogen,
optionally in the form of the individual optical isomers, mixtures of the individual enantiomers or racemates.

7. Use of a compound of general formula **1** according to one of claims 1 to 6 for preparing a medicament for the treatment of diseases in which anticholinergics may develop a therapeutic benefit.

8. Use of a compound of general formula **1** according to one of claims 1 to 6 for preparing a medicament for the treatment of asthma, COPD, vagally induced sinus bradycardia, heart rhythm disorders, spasms in the gastrointestinal tract, spasms in the urinary tract and menstrual disorders.

9. Pharmaceutical preparations containing as active substance one or more compounds of general formula **1** according to one of claims 1 to 6 or the physiologically acceptable salts thereof, optionally combined with conventional excipients and/or carriers.

10. Pharmaceutical preparations according to claim 9, **characterised in that** they contain, in addition to one or more of the compounds of formula **1**, at least one further active substance which is selected from among the betamimetics, antiallergic agents, PAF-antagonists, leukotriene antagonists and steroids.

11. Process for preparing a compound of general formula **1** 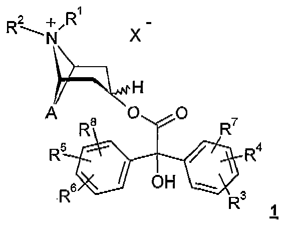 wherein A, X⁻ and the groups R¹, R², R³, R⁴, R⁵, R⁶, R⁷ and R⁸ may have the meanings given in claims 1 to 6, **characterised in that** in a first step a compound of general formula **3** 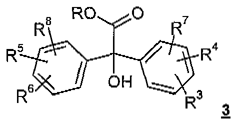 wherein the groups R³, R⁴, R⁵, R⁶, R⁷ and R⁸ may have the meanings given in claims 1 to 6 and R denotes C₁-C₄-alkyl, is reacted with a compound of formula **2** 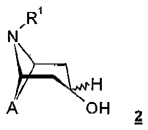 wherein A and R¹ may have the meanings given in claims 1 to 6, to obtain a compound of formula **4** 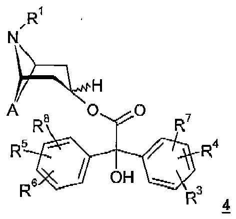 wherein A and the groups R¹, R³, R⁴, R⁵, R⁶, R⁷ and R⁸ may have the meanings given in claims 1 to 6, and this is subsequently quarternised by reacting with a compound R²-X, wherein R² and X may have the meanings given in claims 1 to 6, to obtain a compound of formula **1**.

12. Intermediate products of general formula **4** 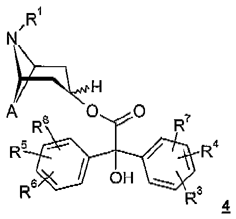 wherein A and the groups R¹, R³, R⁴, R⁵, R⁶, R⁷ and R⁸ may have the meanings given in claims 1 to 6.

## Revendications

1. Composés de formule générale **1** 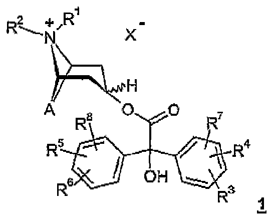 où
A désigne un radical à liaison double choisi dans le groupe comprenant 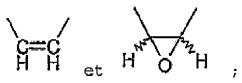
X⁻ désigne un anion simple à charge négative,
R¹ et R² désignent un groupe alkyle en C₁ à C₄ qui peut être substitué éventuellement par un groupe hydroxy ou un atome d'halogène ;
R³, R⁴, R⁵, R⁶, R⁷ et R⁸, identiques ou différents, désignent un atome d'hydrogène ou de fluor, à condition qu'au moins l'un des radicaux R³, R⁴, R⁵, R⁶, R⁷ et R⁸ ne soit pas un atome d'hydrogène,
éventuellement sous la forme des isomères optiques individuels, de mélanges des énantiomères individuels ou de racémates.

2. Composés de formule générale 1 selon la revendication 1,
où
A désigne un radical à liaison double choisi dans le groupe comprenant 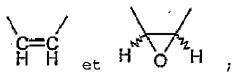
X⁻ désigne un anion simple à charge négative choisi dans le groupe des chlorure, bromure, méthylsulfate, 4-toluéne-sulfonate et méthane-sulfonate ;
R¹ et R², identiques ou différents, désignent un radical choisi dans le groupe comprenant les groupes méthyle, éthyle, n-propyle et iso-propyle, qui peut éventuellement être substitué par un groupe hydroxy ou un atome de fluor ;
R³, R⁴, R⁵, R⁶, R⁷ et R⁸, identiques ou différents, désignent un atome d'hydrogène ou de fluor, à condition qu'au moins l'un des radicaux R³, R⁴, R⁵, R⁶, R⁷ et R⁸ ne soit pas un atome d'hydrogène,
éventuellement sous la forme des isomères optiques individuels, de mélanges des énantiomères individuels ou de racémates.

3. Composés de formule générale 1 selon la revendication 1 ou 2,
où
A désigne un radical à liaison double choisi dans le groupe comprenant 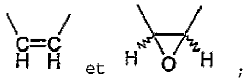
X⁻ désigne un bromure ;
R¹ et R², identiques ou différents, désignent un groupe méthyle ou éthyle ;
R³, R⁴, R⁵, R⁶, R⁷ et R⁸, identiques ou différents, désignent un atome d'hydrogène ou de fluor, à condition qu'au moins l'un des radicaux R³, R⁴, R⁵, R⁶, R⁷ et R⁸ ne soit pas un atome d'hydrogène, éventuellement sous la forme des isomères optiques individuels, de mélanges des énantiomères individuels ou de racémates.

4. Composés de formule générale 1 selon la revendication 1, 2 ou 3,
où
A désigne un radical à liaison double choisi dans le groupe comprenant 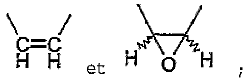
X⁻ désigne un bromure ;
R¹ et R², identiques ou différents, désignent un groupe méthyle ou éthyle ;
R³, R⁴, R⁵, R⁶, R⁷ et R⁸, identiques ou différents, désignent un atome d'hydrogène ou de fluor, à condition qu'au moins 1'un des radicaux R³, R⁴, R⁵, R⁶, R⁷ et R⁸ ne soit pas un atome d'hydrogène, éventuellement sous la forme des isomères optiques individuels, de mélanges des énantiomères individuels ou de racémates.

5. Composés de formule générale 1 selon la revendication 1, 2, 3 ou 4,
où
A désigne un radical à liaison double choisi dans le groupe comprenant 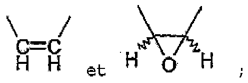
X⁻ désigne un bromure ;
R¹ et R², identiques ou différents, désignent un groupe méthyle ou éthyle ;
R³, R⁴, R⁵, R⁶, R⁷ et R⁸, identiques ou différents, désignent un atome d'hydrogène ou de fluor, à condition qu'au moins l'un des radicaux R³, R⁴, R⁵,
R⁶, R⁷ et R⁸ ne soit pas un atome d'hydrogène, éventuellement sous la forme des isomères optiques individuels, de mélanges des énantiomères individuels ou de racémates.

6. Composés de formule générale **1** selon l'une des revendications 1 à 5,
où
A désigne un radical à liaison double choisi dans le groupe comprenant 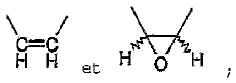
X⁻ désigne un bromure ;
R¹ et R² désignent un groupe méthyle ;
R³, R⁴, R⁵, R⁶, R⁷ et R⁸, identiques ou différents, désignent un atome d'hydrogène ou de fluor, à condition qu'au moins l'un des radicaux R³, R⁴, R⁵, R⁶, R⁷ et R⁸ ne soit pas un atome d'hydrogène,
éventuellement sous la forme des isomères optiques individuels, de mélanges des énantiomères individuels ou de racémates.

7. Utilisation d'un composé de formule générale 1, selon l'une des revendications 1 à 6, pour la production d'un médicament pour le traitement de maladies dans lesquelles des anti-cholinergiques peuvent déployer une utilité thérapeutique.

8. Utilisation d'un composé de formule générale 1 selon l'une des revendications 1 à 6, pour la production d'un médicament pour le traitement de l'asthme, de la BPCO, des bradycardies sinusales d'origine vagale, des troubles du rythme cardiaque, des spasmes des voies gastro-intestinales, des spasmes des voies urinaires et des troubles menstruels.

9. Préparations pharmaceutiques contenant, comme substance active, un ou plusieurs composés de formule générale 1 selon l'une des revendications 1 à 6, ou leurs sels physiologiciquement acceptables, en combinaison avec des adjuvants et/ou des véhicules habituels.

10. Préparations pharmaceutiques selon la revendication 9, **caractérisées en ce que** celles-ci contiennent, parallèlement à un ou plusieurs des composés de formule 1, en outre au moins une autre substance active qui est choisie dans le groupe des bêtamimetiques, des anti-allergiques, des antagonistes du facteur PAF, des antagonistes des leucotriènes et des stéxoïdes.

11. Procédé de production d'un composé de formule générale **1** 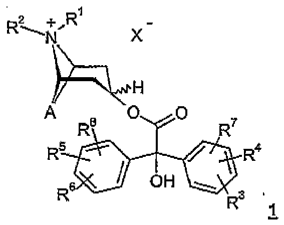 où A, X⁻ et les radicaux R¹, R², R³, R⁴, R⁵, R⁶, R⁷ et R⁸ peuvent avoir les significations indiquées dans les revendications 1 à 6, **caractérisé en ce que**, dans une première étape, on fait réagir un composé de formule générale **3** 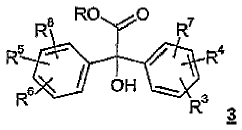 où les radicaux R³, R⁴, R⁵, R⁶, R⁷ et R⁸ peuvent avoir les significations indiquées dans les revendications 1 à 6 et R désigne un groupe alkyle en C₁ à C₄ avec un composé de formule **2** 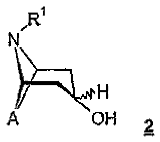 où A et R¹ peuvent avoir les significations indiquées dans les revendications 1 à 6, pour donner un composé de formule **4** 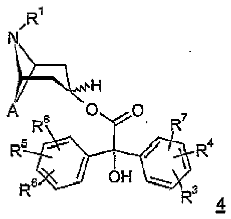 où A et les radicaux R¹, R³, R⁴, R⁵, R⁶, R⁷ et R⁸ peuvent avoir les significations indiquées dans les revendications 1 à 6 et celui-ci est ensuite quaternisé par réaction avec un composé R²X, R² et pouvant avoir les significations mentionnées dans les revendications 1 à 6, en un composé de formule **1**.

12. Produit intermédiaire de formule générale **4** 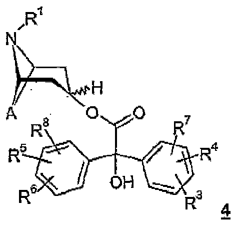 où A et les radicaux R¹, R³, R⁴, R⁵, R⁶, R⁷ et R⁸ peuvent avoir les significations indiquées dans les revendications 1 à 6.
